# EUROPÄISCHE PATENTSCHRIFT

(11) **EP 0 891 341 B1**
(45) Veröffentlichungstag und Bekanntmachung des Hinweises auf die Patenterteilung: **02.01.2003**
(21) Anmeldenummer: 97914253.6
(22) Anmeldetag: 17.03.1997
(51) Int. Cl.: C07D 273/04, C07D 413/06, C07D 498/04, A01N 43/88, C07C 281/02

(54) **1,3,4-OXADIAZIN-DERIVATE UND IHRE VERWENDUNG ALS SCHÄDLINGSBEKÄMPFUNGSMITTEL**
1,3,4-OXADIAZINE DERIVATIVES AND THEIR USE AS PESTICIDES
DERIVES DE 1,3,4-OXADIAZINE ET LEUR UTILISATION COMME PESTICIDES

(30) Priorität: 29.03.1996 DE 19612644
(43) Veröffentlichungstag der Anmeldung: 20.01.1999
(73) Patentinhaber: BAYER AG, 51368 Leverkusen (DE)
(72) Erfinder: DYKER, Hubert, D-51065 Köln (DE); PLANT, Andrew, D-51373 Leverkusen (DE); SCHERKENBECK, Jürgen, D-42929 Wermelskirchen (DE); ERDELEN, Christoph, D-42799 Leichlingen (DE); HARDER, Achim, D-51109 Köln (DE)
(86) Internationale Anmeldenummer: EP9701325
(87) Internationale Veröffentlichungsnummer: WO97036883

(56) Entgegenhaltungen:
- DE-A- 3 627 161
- US-A- 4 670 555
- IL FARMACO, Bd. 50, Nr. 6, Juni 1995, PAVIA IT, Seiten 455-69, XP002032475 J. MARCHAND-BRYNAERT ET AL.: "Design synthesis and evaluation of DD-peptidase and beta-lactamase inhibitors: azapeptides, oxapeptides and related heterocycles"
- CHEMIA ANALITYCZNA, Bd. 17, Nr. 2, 1972, WARSZAWA HU, Seiten 379-85, XP002032476 S. PLAZA: "Jonoforeza bibulowa ketonow i aldehydow w postaci ksanthydrazonow"
- LIEBIGS ANNALEN DER CHEMIE, Nr. 8, 21.August 1981, WEINHEIM DE, Seiten 1433-44, XP002032517 E. FAHR ET AL.: "Azomethin-imine durch Umsetzung von Diphenylketen mit Azodicarbonsäureestern" in der Anmeldung erwähnt

## Beschreibung

Die Erfindung betrifft neue (1,3,4)-Oxadiazin-Derivate, mehrere Verfahren und Zwischenprodukte zu ihrer Herstellung und ihre Verwendung als Schädlingsbekämpfungsmittel, insbesondere als Anthelmintika, Insektizide, Akarizide und Nematizide.

Nur ein einziger Vertreter der 2,5-Dichalkogeno-(1,3,4)-oxadiazinane, das 6,6-Diphenyl-(1,3,4)-oxadiazinan-2,5-dion ist bisher bekannt geworden (Liebigs Ann. Chem. 1981, 1433).

In Chemia Analitycna 17 (1972) 379-385 wird unter anderem die Verbindung HOOC-CH(CH₃)-O-C(=S)NHNH₂ · HCl und ihre Verwendung beim Nachweis von Aldehyden und Ketonen offenbart.

Il Farmaco 50 (6) (1995) 455-469 betrifft das Design, die Synthese und die Bewertung von D,D-Peptidasen und beta-Lactamase-Inhibitoren. Dort werden bestimmte Carbazate sowie ihre Herstellung aus geschützten Vorstufen offenbart.

US 4 670 555 beschreibt substituierte 2,4-Diphenyl-1,3,4-oxadiazin-5-one die unter anderem nematizide Wirkung haben.

Es wurden neue (1,3,4)-Oxadiazin-Derivate der Formel (I) 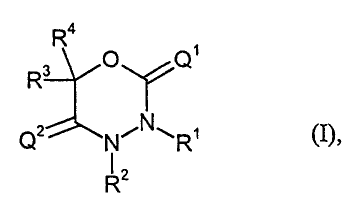 gefunden, in welcher
- R¹ und R²: unabhängig voneinander für Wasserstoff, jeweils gegebenenfalls durch Halogen substituiertes Alkyl, Hydroxyalkyl, Alkanoyloxyalkyl, Alkoxyalkyl, Arylalkoxyalkyl, Mercaptoalkyl, Alkylthioalkyl, Alkylsulfinylalkyl, Alkylsulfonylalkyl, Carboxyalkyl, Alkoxycarbonylalkyl, Aryloxycarbonylalkyl, Arylalkyloxycarbonylalkyl, Carbamoylalkyl, Aminoalkyl, Alkylaminoalkyl, Dialkylaminoalkyl, Alkoxycarbonylaminoalkyl, Alkylcarbonyl, Cycloalkylcarbonyl oder für jeweils gegebenenfalls substituiertes Cycloalkyl, Cycloalkylalkyl, Aryl, Arylalkyl, Arylcarbonyl, Heterocyclylalkyl, Hetaryl oder Hetarylalkyl stehen oder
- R¹, R²: und die beiden Stickstoffatome an die sie gebunden sind für einen gegebenenfalls substituierten heterocyclischen Ring stehen,
- R³ und R⁴: unabhängig voneinander für Wasserstoff, jeweils gegebenenfalls durch Halogen substituiertes Alkyl, Alkenyl, Hydroxyalkyl, Alkanoyloxyalkyl, Alkoxyalkyl, Arylalkoxyalkyl, Mercaptoalkyl, Alkylthioalkyl, Alkylsulfinylalkyl, Alkylsulfonylalkyl, Alkoxycarbonylalkyl, Alkoxycarbonylalkyl, Arylalkyloxycarbonylalkyl, Carbamoylalkyl, Aminoalkyl, Alkylaminoalkyl, Dialkylaminoalkyl, Alkylcarbonyl, Cycloalkylcarbonyl oder für jeweils gegebenenfalls substituiertes Cycloalkyl, Cycloalkylalkyl, Aryl, Arylalkyl, Hetaryl oder Hetarylalkyl stehen oder
- R³ und R⁴: gemeinsam für Alkylen oder den Rest (a) 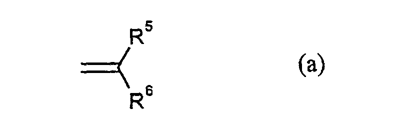 stehen, worin
R⁵ und R⁶ unabhängig voneinander für Wasserstoff, jeweils gegebenenfalls durch Halogen substituiertes Alkyl, Alkenyl, Hydroxyalkyl, Alkanoyloxyalkyl, Alkoxyalkyl, Arylalkoxyalkyl, Mercaptoalkyl, Alkylthioalkyl, Alkylsulfinylalkyl, Alkylsulfonylalkyl, Alkoxycarbonylalkyl, Aryloxycarbonylalkyl, Aminoalkyl, Alkylaminoalkyl, Dialkylaminoalkyl, Alkylcarbonyl oder für jeweils gegebenenfalls substituiertes Cycloalkyl, Cycloalkylalkyl, Aryl, Arylalkyl, Hetaryl oder Hetarylalkyl stehen und
- Q¹ und Q²: unabhängig voneinander für Sauerstoff oder Schwefel stehen,
wobei 6,6-Diphenyl-(1,3,4)-oxadiazinan-2,5-dion ausgenommen ist.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Weiterhin wurde gefunden, daß man die neuen Verbindungen der Formel (I) nach einem der im folgenden beschriebenen Verfahren erhält.
A) (1,3,4)-Oxadiazin-Derivate der Formel (I-a) 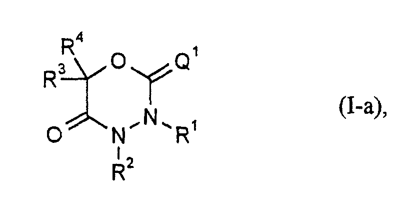 in welcher
   - R¹ bis R⁴ und Q¹: die oben angegebenen Bedeutungen haben,
   lassen sich herstellen, indem man Carbazate der Formel (II) 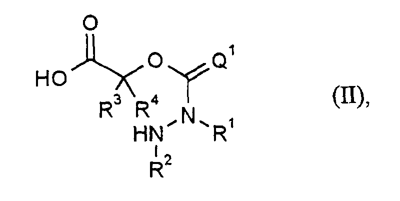 in welcher
   - R¹ bis R⁴ und Q¹: die oben angegebenen Bedeutungen haben,
   in Gegenwart eines Reaktionshilfsmittels und eines Verdünnungsmittels und gegebenenfalls in Gegenwart einer Base umsetzt.
B) (1,3,4)-Oxadiazin-Derivate der Formel (I-b) 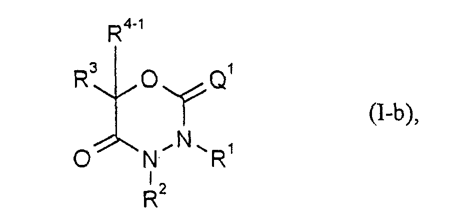 in welcher
   - R¹ bis R³, Q¹ und Q²: die oben angegebenen Bedeutungen haben und
   - R⁴⁻¹: mit Ausnahme von Wasserstoff für dieselben Reste wie R⁴ steht,
   lassen sich herstellen, indem man
   (1,3,4)-Oxadiazin-Derivate der Formel (I-c) 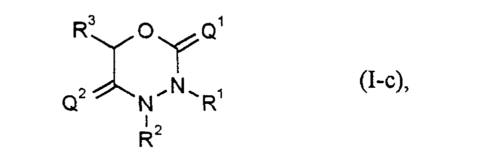 in welcher
   - R¹ und R²: andere Bedeutungen als Wasserstoff haben,
   - R³, Q¹ und Q²: die oben angegebenen Bedeutungen haben
   mit Verbindungen der Formel (III)

   R⁴⁻¹―E (III),

   in welcher
   - R⁴⁻¹: die oben angegebene Bedeutung hat und
   - E: für eine elektronenziehende Abgangsgruppe steht,
   gegebenenfalls in Gegenwart eines Verdünnungsmittels und gegebenenfalls in Gegenwart eines Reaktionshilfsmittels umsetzt.
C) (1,3,4)-Oxadiazin-Derivate der Formel (I-d) 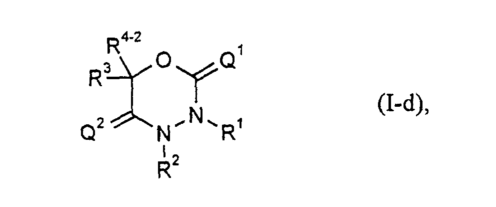 in welcher
   - R¹ bis R³, Q¹ und Q²: die oben angegebenen Bedeutungen haben und
   - R⁴⁻²: für den Rest (b) 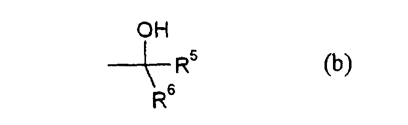 steht, worin
   R⁵ und R⁶ unabhängig voneinander für Wasserstoff, gegebenenfalls substituiertes Alkyl oder Aryl steht, oder
   - R³ und R⁴⁻²: gemeinsam für den Rest (a) 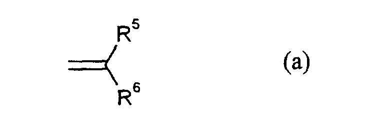 stehen, worin
   R⁵ und R⁶ die oben angegebenen Bedeutungen haben, lassen sich herstellen, indem man
   (1,3,4)-Oxadiazin-Derivate der Formel (I-c) 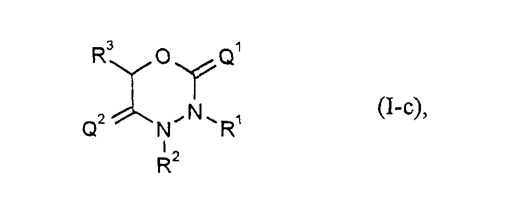 in welcher
   R¹ bis R³, Q¹ und Q² die oben angegebenen Bedeutungen haben mit Ketonen oder Aldehyden der Formel (IV)

   R⁵―CO―R⁶ (IV),

   in welcher
   R⁵ und R⁶ die oben angegebenen Bedeutungen haben, gegebenenfalls in Gegenwart eines Verdünnungsmittels und gegebenenfalls in Gegenwart eines Reaktionshilfsmittels umsetzt und gegebenenfalls anschließend Wasser eliminiert.
D) (1,3,4)-Oxadiazin-Derivate der Formel (I-e) 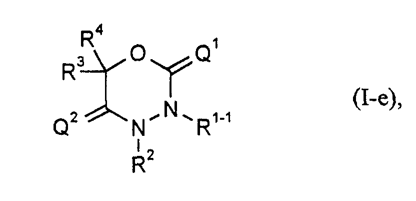 in welcher
   - R¹⁻¹: mit Ausnahme von Wasserstoff für dieselben Reste wie R¹ steht,
   - R² bis R⁴, Q¹ und Q²: die oben angegebenen Bedeutungen haben,
   lassen sich herstellen, indem man (1,3,4)-Oxadiazin-Derivate der Formel (I-f) 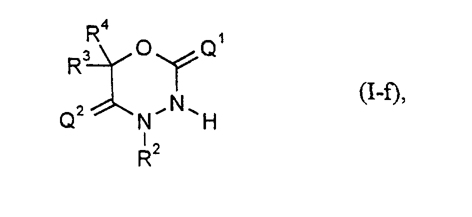 in welcher
   - R² bis R⁴, Q¹ und Q²: die oben angegebenen Bedeutungen haben
   mit Verbindungen der Formel (V)

   R¹⁻¹―E (V)

   in welcher
   - R¹⁻¹: die oben angegebene Bedeutung hat und
   - E: für eine elektronenziehende Abgangsgruppe steht,
   gegebenenfalls in Gegenwart eines Verdünnungsmittels und gegebenenfalls in Gegenwart eines Reaktionshilfsmittels umsetzt.
E) (1,3,4)-Oxadiazin-Derivate der Formel (I-g) 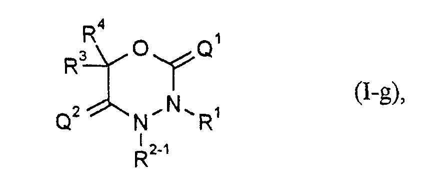 in welcher
   - R¹, R³, R⁴, Q¹ und Q²: die oben angegebenen Bedeutungen haben und
   - R²⁻¹: mit Ausnahme von Wasserstoff für dieselben Reste wie R² steht,
   lassen sich herstellen, indem man (1,3,4)-Oxadiazin-Derivate der Formel (I-h) 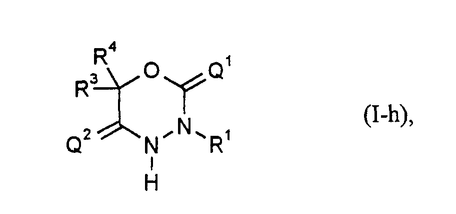 in welcher
   - R¹, R³, R⁴, Q¹ und Q²: die oben angegebenen Bedeutungen haben
   mit Verbindungen der Formel (VI)

   R²⁻¹―E (VI),

   in welcher
   - R²⁻¹: die oben angegebene Bedeutung hat und
   - E: für eine elektronenziehende Abgangsgruppe steht,
   gegebenenfalls in Gegenwart eines Verdünnungsmittels und gegebenenfalls in Gegenwart eines Reaktionshilfsmittels umsetzt.
F) (1,3,4)-Oxadiazin-Derivate der Formel (I) 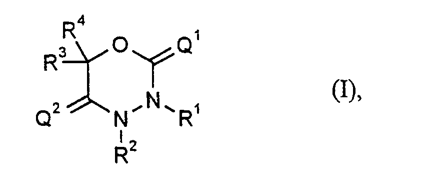 in welcher
   - R¹ bis R⁴, Q¹ und Q²: die oben angegebenen Bedeutungen haben,
   lassen sich herstellen, indem man Verbindungen der Formel (VII) 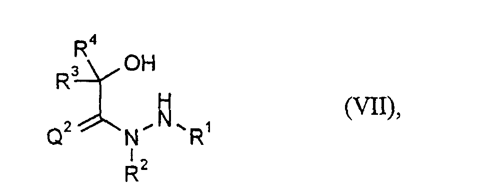 in welcher
   - R¹ bis R⁴ und Q²: die oben angegebenen Bedeutungen haben,
   mit Verbindungen der Formel (VIII) 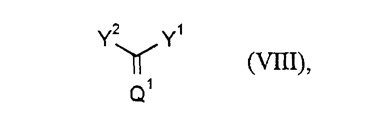 in welcher
   - Y¹: für Chlor, Trichlormethoxy, C₁-C₄-Alkoxy, gegebenenfalls substituiertes Phenoxy, 1-Imidazolyl oder 1,2,4-Triazolyl steht und
   - Y²: für Chlor, Trichlormethoxy, 1-Imidazolyl oder 1,2,4-Triazolyl steht,
   - Q¹: die oben angegebene Bedeutung hat,
   gegebenenfalls in Gegenwart eines Verdünnungsmittels und gegebenenfalls in Gegenwart eines Reaktionshilfsmittels umsetzt und cyclokondensiert.
G) (1,3,4)-Oxadiazin-Derivate der Formel (I) 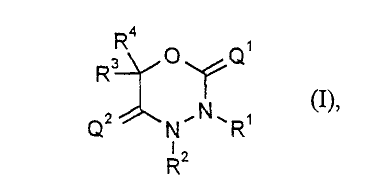 in welcher
   - R¹ bis R⁴, Q¹ und Q²: die oben angegebenen Bedeutungen haben,
   lassen sich herstellen, indem man Verbindungen der Formel (IX) 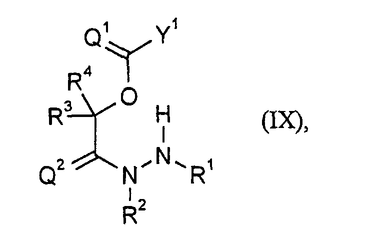 in welcher
   - R¹ bis R⁴, Q¹, Q² und Y¹: die oben angegebenen Bedeutungen haben,
   gegebenenfalls in Gegenwart eines Verdünnungsmittels und gegebenenfalls in Gegenwart eines Reaktionshilfsmittels cyclokondensiert.
H) (1,3,4)-Oxadiazin-Derivate der Formel (I-i) 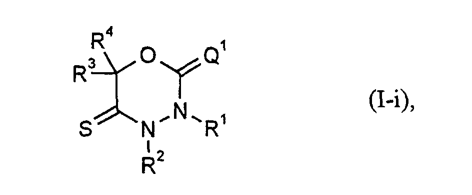 in welcher
   - R¹ bis R⁴ und Q¹: die oben angegebenen Bedeutungen haben,
   lassen sich herstellen, indem man (1,3,4)-Oxadiazin-Derivate der Formel (I-a) 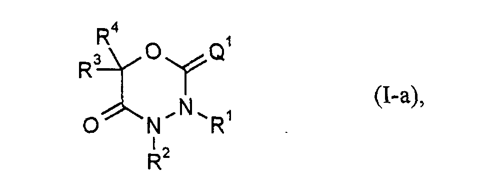 in welcher
   - R¹ bis R⁴ und Q¹: die oben angegebenen Bedeutungen haben,
   mit einem Thionylierungsreagenz gegebenenfalls in Gegenwart eines Verdünnungsmittels umsetzt.

Weiterhin wurde gefunden, daß die neuen Verbindungen der Formel (I) eine sehr gute Wirksamkeit als Schädlingsbekämpfungsmittel, vorzugsweise zur Bekämpfung von Endoparasiten bei Nutztieren und von Insekten, Spinnentieren und Nematoden, die in Landwirtschaft, in Forsten, im Vorrats- und Materialschutz sowie auf dem Hygienesektor vorkommen, aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert.
- R¹ und R²: stehen unabhängig voneinander bevorzugt für Wasserstoff, C₁-C₁₅-Alkyl, insbesondere auch 3,7,11-Trimethyldodecyl, für jeweils gegebenenfalls durch Fluor, Chlor oder Brom substituiertes C₁-C₈-Alkyl, C₃-C₇-Cycloalkyl, C₃-C₇-Cycloalkyl-C₁-C₄-alkyl, C₁-C₆-Hydroxyalkyl, C₁-C₄-Alkanoyloxy-C₁-C₆-alkyl, C₁-C₄-Mkoxy-C₁-C₆-alkyl, Aryl-C₁-C₄-alkoxy-C₁-C₆-alkyl, C₁-C₆-Mercaptoalkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, Carboxy-C₁-C₆-alkyl, insbesondere Carboxymethyl oder Carboxyethyl, C₁-C₄-Alkoxycarbonyl-C₁-C₆-alkyl, insbesondere Methoxycarbonylmethyl oder Ethoxycarbonylethyl, Aryloxycarbonyl-C₁-C₆-alkyl, insbesondere Phenoxycarbonylmethyl, Aryl-C₁-C₄-alkyloxycarbonyl-C₁-C₆-alkyl, insbesondere Benzyloxycarbonylmethyl, Carbamoyl-C₁-C₆-alkyl, insbesondere Carbamoylmethyl oder Carbamoylethyl, Amino-C₁-C₆-alkyl, insbesondere Aminopropyl oder Aminobutyl, C₁-C₄-Alkylamino-C₁-C₆-alkyl insbesondere Methylaminopropyl oder Methylamino, Di-(C₁-C₄)-alkylamino-C₁-C₆-alkyl insbesondere Dimethylaminopropyl oder Dimethylaminobutyl, C₁-C₄-Alkoxycarbonylamino-C₁-C₆-alkyl, C₁-C₆-Alkylcarbonyl, C₃-C₇-Cycloalkylcarbonyl oder für jeweils gegebenenfalls durch Halogen, Hydroxy, Nitro, Cyano, Amino, C₁-C₄-Alkylamino, Di-(C₁-C₄)-alkylamino, Benzylamino, Dibenzylamino, geschütztes Amino wie z.B. Acetyl-, t-Butoxycarbonyl-, Benzyloxycarbonyl- oder FMOC-amino, C₁-C₆-Alkyl, C₁-C₄-Halogenalkyl, C₁-C₄-Alkoxy oder C₁-C₄-Halogenalkoxy substituiertes Aryl, Aryl-C₁-C₄-alkyl, Arylcarbonyl, Aryl-C₁-C₄-Alkylcarbonyl, Hetaryl oder Hetaryl-C₁-C₄-alkyl, wobei gegebenenfalls eine NH-Funktion im heterocyclischen Ring durch eine Aminoschutzgruppe, wie beispielhaft o.a., derivatisiert sein kann. sowie gegebenenfalls C₁-C₄-alkylsubstituiertes Heterocyclyl-C₁-C₄-alkyl wie z.B. Dioxolanylmethyl.
- R¹ und R²: stehen gemeinsam bevorzugt mit den beiden Stickstoffatomen, an die sie gebunden sind, für einen gegebenenfalls durch Halogen, Hydroxy, Nitro, Cyano, C₁-C₄-Alkyl, C₁-C₄-Halogenalkyl, C₁-C₄-Alkoxy oder C₁-C₄-Halogenalkoxy substituierten 5- bis 8-gliedrigen gesättigten oder ungesättigten heterocyclischen Ring.
- R³ und R⁴: stehen unabhängig voneinander bevorzugt für Wasserstoff, C₁-C₁₆-Alkyl, für jeweils gegebenenfalls durch Fluor, Chlor oder Brom substituiertes C₁-C₆-Alkyl,- C₂-C₈-Alkenyl, C₃-C₇-Cycloalkyl, C₃-C₇-Cycloalkyl-C₁-C₄-alkyl, C₁-C₆-Hydroxyalkyl, C₁-C₄-Alkanoyloxy-C₁-C₆-alkyl insbesondere Acetoxymethyl oder 1-Acetoxyethyl, C₁-C₄-Alkoxy-C₁-C₆-alkyl insbesondere Methoxymethyl oder 1-Methoxyethyl, Aryl-C₁-C₄-alkoxy-C₁-C₆-alkyl, C₁-C₆-Mercaptoalkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, C₁-C₄-Alkoxycarbonyl-C₁-C₆-alkyl, Aryloxycarbonyl-C₁-C₆-alkyl, insbesondere Phenoxycarbonylmethyl, Aryl-C₁-C₄-alkyloxycarbonyl-C₁-C₆-alkyl, Carbamoyl-C₁-C₆-alkyl, insbesondere Carbamoylmethyl oder Carbamoylethyl, Amino-C₁-C₆-alkyl, C₁-C₄-Alkylamino-C₁-C₆-alkyl, Di-C₁-C₄-alkylamino-C₁-C₆-alkyl, C₁-C₆-Alkylcarbonyl, C₃-C₇-Cycloalkylcarbonyl, oder für jeweils gegebenenfalls durch Halogen, Hydroxy, Cyano, C₁-C₆-Alkyl, C₁-C₄-Halogenalkyl, C₁-C₄-Alkoxy, C₁-C₄-Halogenalkoxy, Benzyloxy oder Silyloxy, das durch C₁-C₄-Alkyl und/oder Phenyl trisubstituiert ist, substituiertes Aryl, Aryl-C₁-C₄-alkyl, Arylcarbonyl, Hetaryl oder Hetaryl-C₁-C₄-alkyl.
- R³ und R⁴: stehen gemeinsam bevorzugt für C₂-C₇-Alkylen oder den Rest (a) 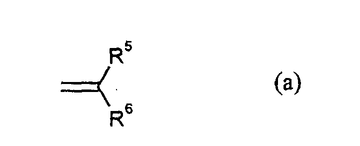 worin
R⁵ und R⁶ unabhängig voneinander für Wasserstoff, jeweils gegebenenfalls durch Fluor, Chlor oder Brom substituiertes C₁-C₆-Alkyl, C₂-C₄-1-Alkenyl oder C₃-C₇-Cycloalkyl oder für jeweils gegebenenfalls durch Halogen, Hydroxy, Nitro, C₁-C₆-Alkyl, C₁-C₄-Halogenalkyl, C₁-C₄-Alkoxy, C₁-C₄-Halogenalkoxy, Phenoxy, Amino, C₁-C₄-Alkylamino oder Di-(C₁-C₄)-alkylamino, substituiertes Phenyl, Phenyl-C₁-C₄-alkyl oder 5- oder 6-gliedriges Hetaryl stehen.
Q¹ und Q² stehen unabhängig voneinander bevorzugt für Sauerstoff oder Schwefel.

Aus dem bevorzugten Bereich ausgenommen ist 6,6-Diphenyl-(1,3,4)-oxadiazinan-2,5-dion.
- R¹ und R²: stehen unabhängig voneinander besonders bevorzugt für Wasserstoff, C₁-C₁₀-Alkyl, für jeweils gegebenenfalls durch Fluor, Chlor oder Brom substituiertes C₁-C₄-Alkyl, C₃-C₇-Cycloalkyl, insbesondere Cyclopentyl, Cyclohexyl oder Cycloheptyl, C₃-C₇-Cycloalkyl-C₁-C₄-alkyl, C₁-C₆-Hydroxyalkyl, insbesondere Hydroxymethyl oder 1-Hydroxyethyl, C₁-C₄-Alkanoyloxy-C₁-C₆-alkyl, insbesondere Acetoxymethyl oder 1-Acetoxyethyl, C₁-C₄-Alkoxy-C₁-C₆-alkyl, insbesondere Methoxymethyl oder 1-Methoxyethyl, Phenyl-C₁-C₄-alkoxy-C₁-C₆-alkyl, insbesondere Benzyloxymethyl oder 1-Benzyloxyethyl, C₁-C₄-Alkoxycarbonylamino-C₁-C₆-alkyl, insbesondere tert.-Butoxycarbonylaminopropyl oder tert.-Butoxycarbonylaminobutyl, C₁-C₆-Alkylcarbonyl, insbesondere Acetyl, Propionyl oder Butyryl, C₃-C₇-Cycloalkylcarbonyl, insbesondere Cyclopropylcarbonyl oder Cyclohexylcarbonyl oder für jeweils gegebenenfalls durch Fluor, Chlor, Brom, Iod, Hydroxy, Nitro, Cyano, Amino, C₁-C₄-Alkylamino, Di-(C₁-C₄)-alkylamino, Benzylamino, Dibenzylamino, geschütztes Amino wie z.B. Acetyl-, t-Butoxycarbonyl-, Benzyloxycarbonyl- oder FMOC-amino, C₁-C₄-Alkyl, C₁-C₄-Halogenalkyl, C₁-C₄-Alkoxy oder C₁-C₂-Halogenalkoxy substituiertes Phenyl, Phenyl-C₁-C₄-alkyl, Phenylcarbonyl, 5- oder 6-gliedriges Hetaryl, insbesondere Thienyl, Thiazolyl oder Pyridyl, 5- oder 6-gliedriges Hetaryl-C₁-C₄-alkyl oder Indolyl-C₁-C₄-alkyl.
- R¹ und R²: stehen gemeinsam besonders bevorzugt mit den beiden Stickstoffatomen, an die sie gebunden sind, für einen gegebenenfalls durch Fluor, Chlor, Brom, Hydroxy, Nitro, Cyano, C₁-C₄-Alkyl, C₁-C₄-Halogenalkyl, C₁-C₄-Alkoxy oder C₁-C₂-Halogenalkoxy substituierten 5- bis 8-gliedrigen gesättigten oder ungesättigten heterocyclischen Ring.
- R³ und R⁴: stehen unabhängig voneinander besonders bevorzugt für Wasserstoff, C₁-C₁₂-Alkyl, für jeweils gegebenenfalls durch Fluor, Chlor oder Brom substituiertes C₁-C₄-Alkyl, C₂-C₆-Alkenyl, C₃-C₇-Cycloalkyl, insbesondere Cyclopentyl, Cyclohexyl oder Cycloheptyl, C₃-C₇-Cycloalkyl-C₁-C₄-alkyl, C₁-C₆-Hydroxyalkyl, Phenyl-C₁-C₄-alkoxy-C₁-C₆-alkyl, insbesondere Benzyloxymethyl oder 1-Benzyloxyethyl, C₁-C₄-Alkylthio-C₁-C₆-alkyl, insbesondere Methylthioethyl, C₁-C₄-Alkoxycarbonyl-C₁-C₆-alkyl, insbesondere Methoxycarbonylmethyl oder Ethoxycarbonylethyl, Phenyl-C₁-C₄-alkyloxycarbonyl-C₁-C₆-alkyl, insbesondere Benzyloxycarbonylmethyl, Amino-C₁-C₆-alkyl, insbesondere Aminopropyl oder Aminobutyl, C₁-C₄-Alkylamino-C₁-C₆-alkyl insbesondere Methylaminopropyl oder Methylamino, Di-(C₁-C₄)-alkylamino-C₁-C₆-alkyl insbesondere Dimethylaminopropyl oder Dimethylaminobutyl, C₁-C₆-Alkylcarbonyl, insbesondere Acetyl, Propionyl oder Butyryl, C₃-C₇-Cycloalkylcarbonyl, insbesondere Cyclopropylcarbonyl oder Cyclohexylcarbonyl oder für jeweils gegebenenfalls durch Fluor, Chlor, Brom, Iod, Hydroxy, Cyano, C₁-C₆-Alkyl, C₁-C₄-Halogenalkyl, C₁-C₄-Alkoxy, C₁-C₄-Halogenalkoxy, Benzyloxy oder Silyloxy, das durch C₁-C₄-Alkyl und/oder Phenyl trisubstituiert ist, substituiertes Phenyl, Phenyl-C₁-C₄-alkyl, Naphthylmethyl, Phenylcarbonyl, 5- oder 6-gliedriges Hetaryl, insbesondere Thienyl, Thiazolyl oder Pyridyl, Indolyl, Benzo-1,3-dioxolyl, 5- oder 6-gliedriges Hetaryl-C₁-C₄-alkyl insbesondere Thienylmethyl, Thiazolylmethyl, Imidazolylmethyl oder Pyridylmethyl oder Indolyl-C₁-C₄-alkyl.
- R³ und R⁴: stehen gemeinsam besonders bevorzugt für C₂-C₆-Alkylen oder den Rest (a) 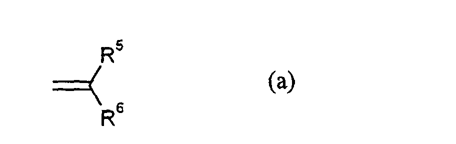 worin
R⁵ und R⁶ unabhängig voneinander für Wasserstoff, jeweils gegebenenfalls durch Fluor, Chlor oder Brom substituiertes C₁-C₄-Alkyl oder C₃-C₆-Cycloalkyl, insbesondere Cyclopropyl, Cyclopentyl oder Cyclohexyl oder für jeweils gegebenenfalls durch Fluor, Chlor, Brom, Hydroxy, Nitro, C₁-C₄-Mkyl, C₁-C₄-Halogenalkyl, C₁-C₄-Alkoxy, C₁-C₄-Halogenalkoxy, Phenoxy, Amino, C₁-C₄-Alkylamino, insbesondere Methylamino oder Ethylamino oder Di-(C₁-C₄)-alkylamino, insbesondere Dimethylamino oder Diethylamino substituiertes Phenyl, Phenyl-C₁-C₄-alkyl oder 5- oder 6-gliedriges Hetaryl stehen.
- Q¹: steht besonders bevorzugt für Sauerstoff oder Schwefel.
- Q²: steht besonders bevorzugt für Sauerstoff.

Aus dem besonders bevorzugten Bereich ausgenommen ist 6,6-Diphenyl-(1,3,4)-oxadiazinan-2,5-dion.
- R¹ und R²: stehen unabhängig voneinander ganz besonders bevorzugt für Wasserstoff, C₁-C₁₀-Alkyl, insbesondere Methyl, Ethyl, n-Propyl, Isopropyl, n-Butyl, Isobutyl, sec.-Butyl, tert.-Butyl, n-Pentyl, Isopentyl, sec.-Pentyl, tert.-Pentyl, n-Hexyl, Isohexyl, sec.-Hexyl, n-Heptyl, Isoheptyl, sec.-Heptyl, Oktyl, Isooctyl, sec.-Octyl oder 3,7-Dimethyloctyl, für C₃-C₇-Cycloalkyl-C₁-C₄-alkyl inbesondere Cyclopentylmethyl, Cyclohexylmethyl oder Cycloheptylmethyl, für jeweils gegebenenfalls durch Fluor, Chlor, Brom, Iod, Hydroxy, Cyano, Methyl, Ethyl, n-Propyl, Isopropyl, n-Butyl, Isobutyl, sec.-Butyl, tert.-Butyl, Trifluormethyl, Trichlormethyl, Methoxy, Difluormethoxy, Trifluormethoxy oder Benzyloxy substituiertes Phenyl, Benzyl, Phenethyl, 5- oder 6-gliedriges Hetarylmethyl, insbesondere Thienylmethyl, Thiazolylmethyl, Furylmethyl oder Pyridylmethyl oder für Indolylmethyl.
- R¹ und R²: stehen gemeinsam ganz besonders bevorzugt mit den beiden Stickstoffatomen, an die sie gebunden sind, für einen 5- oder 6-gliedrigen gesättigten oder ungesättigten heterocyclischen Ring, insbesondere für -(CH₂)₃- und -(CH₂)₄-.
- R³ und R⁴: stehen unabhängig voneinander ganz besonders bevorzugt für Wasserstoff, C₁-C₁₂-Alkyl, insbesondere Methyl, Ethyl, n-Propyl, Isopropyl, n-Butyl, Isobutyl, sec.-Butyl, tert.-Butyl, n-Pentyl, Isopentyl, sec.-Pentyl, tert.-Pentyl, n-Hexyl, Isohexyl, sec.-Hexyl, n-Heptyl, Isoheptyl, sec.-Heptyl, Oktyl, Isooctyl, sec.-Octyl, n-Decyl oder n-Dodecyl, für jeweils gegebenenfalls durch Fluor oder Chlor substituiertes C₁-C₄-Alkyl, insbesondere Fluormethyl, Trifluormethyl oder Trichlormethyl, für C₂-C₆-Alkenyl, insbesondere Vinyl oder Allyl, für Cyclopentyl oder Cyclohexyl, für C₃-C₇-Cycloalkyl-C₁-C₄-alkyl, insbesondere Cyclopropylmethyl, für Methylthioethyl oder für jeweils gegebenenfalls durch Fluor, Chlor, Brom, Iod, Hydroxy, Cyano, Methyl, Ethyl, n-Propyl, Isopropyl, n-Butyl, Isobutyl, sec.-Butyl, tert.-Butyl, Trifluormethyl, Trichlormethyl, Methoxy, Difluormethoxy, Trifluormethoxy oder Benzyloxy substituiertes Phenyl, Phenyl-C₁-C₄-alkyl, insbesondere Benzyl, 3-Naphthylmethyl, Benzo-1,3-dioxol-5-yl, Thienylmethyl, Imidazolylmethyl oder Indolylmethyl.
- R³ und R⁴: stehen gemeinsam ganz besonders bevorzugt für -(CH₂)₂-, -CH(CH₃)CH₂-, -C(CH₃)₂CH₂-, -(CH₂)₃-, -(CH₂)₄-, -(CH₂)₅- oder den Rest (a) 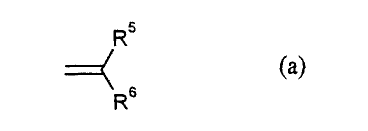 worin
R⁵ und R⁶ unabhängig voneinander für Wasserstoff, für gegebenenfalls durch Fluor oder Chlor substituiertes Methyl, Ethyl, n-Propyl, Isopropyl, n-Butyl, Isobutyl, sec.-Butyl oder tert.-Butyl oder für jeweils gegebenenfalls durch Fluor, Chlor, Brom, Nitro, Amino, Methyl, Ethyl, Trifluormethyl, Methoxy, Difluormethoxy oder Trifluormethoxy substituiertes Phenyl, Benzyl oder Pyridyl stehen.

Aus dem ganz besonders bevorzugten Bereich ausgenommen ist 6,6-Diphenyl-(1,3,4)-oxadiazinan-2,5-dion.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Verwendet man beispielsweise 3-Methoxy-2-methyl-2-(tetrahydropyridazin-1-carbothionyloxy)-propionsäure als Ausgangsstoff, so kann der Reaktionsablauf des erfindungsgemäßen Verfahrens (A) durch das folgende Formelschema wiedergegeben werden:

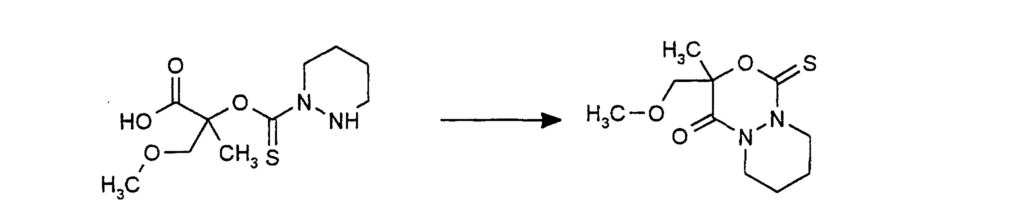

Verwendet man beispielsweise 3-tert.-Butyl-4-(2-thienylmethyl)-2,5-dioxo-(1,3,4)-oxadiazin und Benzylbromid als Ausgangsstoffe, so kann der Reaktionsablauf des erfindungsgemäßen Verfahrens (B) durch das folgende Formelschema wieder gegeben werden:

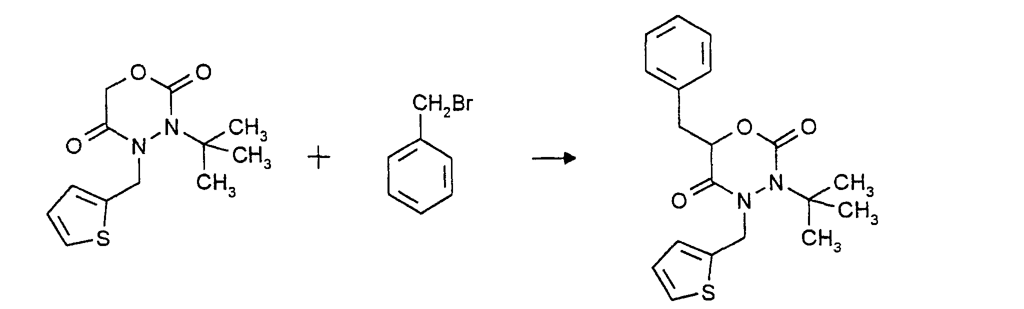

Verwendet man beispielsweise 3,4-Dimethyl-2,5-dioxo-(1,3,4)-oxadiazin und Aceton als Ausgangsstoffe, so kann der Reaktionsablauf des erfindungsgemäßen Verfahrens (C) durch das folgende Formelschema wiedergegeben werden:

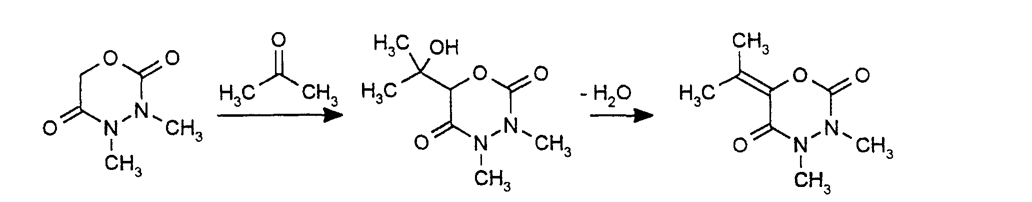

Verwendet man beispielsweise 4,6-Dimethyl-6-vinyl-2,5-dioxo-(1,3,4)-oxadiazin und Iodmethan als Ausgangsstoffe, so kann der Reaktionsablauf des erfindungsgemäßen Verfahrens (D) durch das folgende Formelschema wiedergegeben werden:

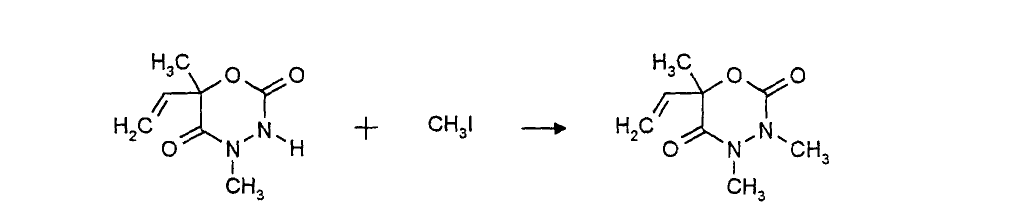

Verwendet man beispielsweise 3-Methyl-6-(2-methylpropyl)-2,5-dioxo-(1,3,4)-oxadiazin und Dimethylsulfat als Ausgangsstoffe, so kann der Reaktionsablauf des erfindungsgemäßen Verfahrens (E) durch das folgende Formelschema wiedergegeben werden:

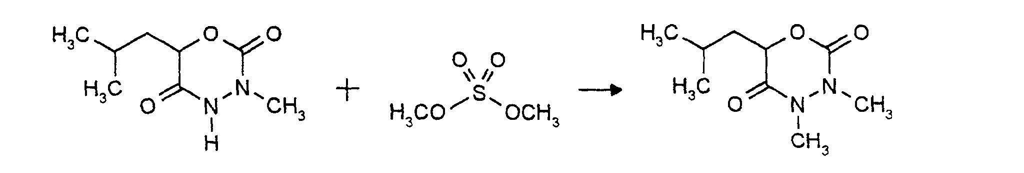

Verwendet man beispielsweise 1-Hydroxy-2-methyl-cyclopropancarbonsäure-2-[(4-chlorphenyl)-1-methyl-hydrazid] und Phosgen als Ausgangsstoffe, so kann der Reaktionsablauf des erfindungsgemäßen Verfahrens (F) durch das folgende Formelschema wiedergegeben werden:

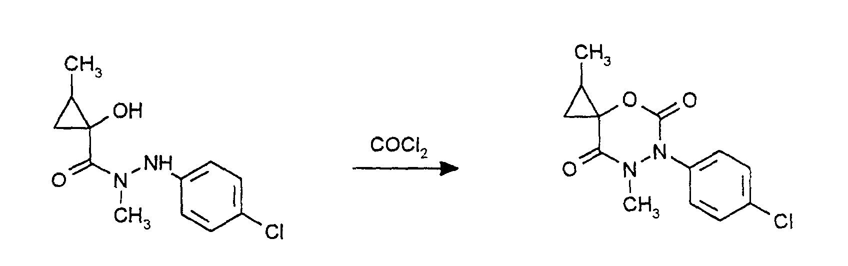

Verwendet man beispielsweise 3-Methyl-2-(4-nitrophenoxycarbonyloxy)-buttersäure-(1-phenyl-2-methyl-hydrazid) als Ausgangsstoff, so kann der Reaktionsablauf des erfindungsgemäßen Verfahrens (G) durch das folgende Formelschema wiedergegeben werden:

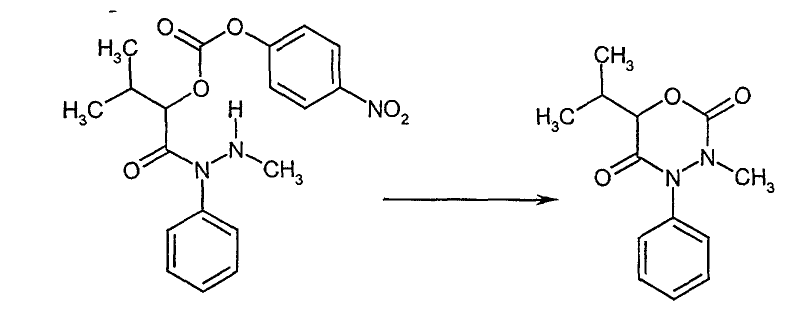

Verwendet man beispielsweise 4-(4-Methylpentyl)-3-methyl-6-phenyl-2,5-dioxo-(1,3,4)-oxadiazin und [2,4-Bis(4-methoxyphenyl)-1,3,2,4-dithiadiphosphetan-2,4-dithion (Lawesson-Reagenz) als Ausgangsstoffe, so kann der Reaktionsablauf des erfindungsgemäßen Verfahrens (H) durch das folgende Formelschema wiedergegeben werden:

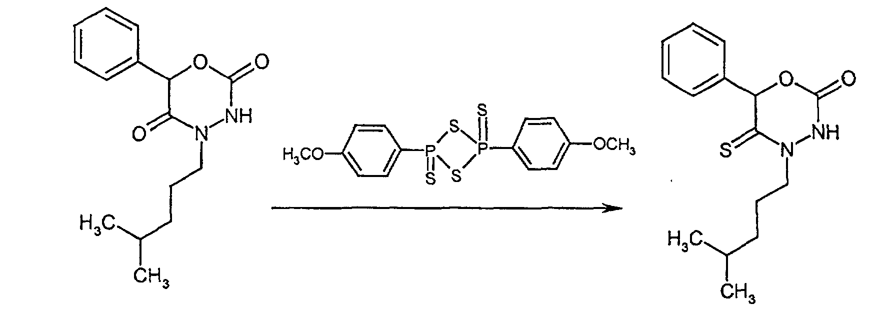

Die zur Durchführung des erfindungsgemäßen Verfahrens (A) benötigten Carbazate sind durch die Formel (II) allgemein definiert. In dieser Formel stehen R¹, R², R³, R⁴ und Q¹ vorzugsweise für diejenigen Reste, die bereits im Zusammenhang mit der Beschreibung der Oxadiazin-Derivate der Formel (I) als bevorzugte Substituenten genannt wurden. Die Carbazate der Formel (II) sind neu, mit der Ausnahme der Verbindungen, in denen gleichzeitig R² und R⁴ jeweils für Wasserstoff, R¹ für Wasserstoff oder C₁-C₅-Alkyl und R³ für Methyl oder Benzyl stehen, sowie weiterhin mit Ausnahme der Verbindungen, in denen R¹ für Methyl, R² für Phenylcarbonyl, R³ für Methyl oder Phenyl, R⁴ für Wasserstoff und Q¹ für Sauerstoff steht (vgl. DE-OS 2 658 254, Chemia Analityczna 17 (1972) 379-385 und Il Farmaco 50, (6) (1995) 455-469.

Carbazate der Formel (II) lassen sich z.B. herstellen, indem man in einem Verfahren (A.a) die Schutzgruppe A² C-terminal geschützter Carbazate der Formel (X) gemäß dem folgende Reaktionsschema abspaltet:

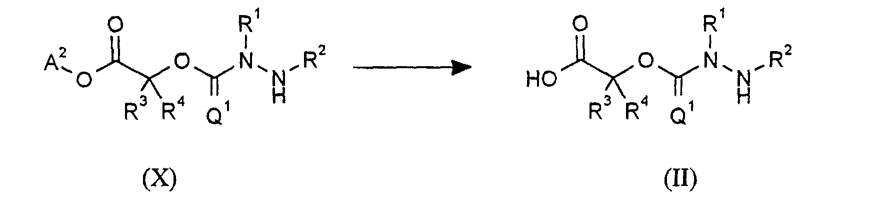

In Formel (XI) steht A² für eine C-terminale Schutzgruppe wie beispielsweise tert.-Butyl oder Benzyl (Vgl. z.B. T. W. Greene, P. G. M. Wuts, Protective Groups in Organic Synthesis, 2. Ed., John Wiley & Sons, New York 1991).

Die Reaktion läßt sich mittels üblicher Methoden C-terminaler Deblockierung wie Acidolyse, beispielsweise im Falle eines tert.-Butylesters, oder katalytischer Hydrierung, beispielsweise im Falle eines Benzylesters, durchführen.

Carbazate der Formel (II) lassen sich z.B. auch herstellen, indem man in einem Verfahren (A.b) die Schutzgruppe A³ N-terminal geschützter Carbazate der Formel (XI) gemäß dem folgenden Reaktionsschema abspaltet:

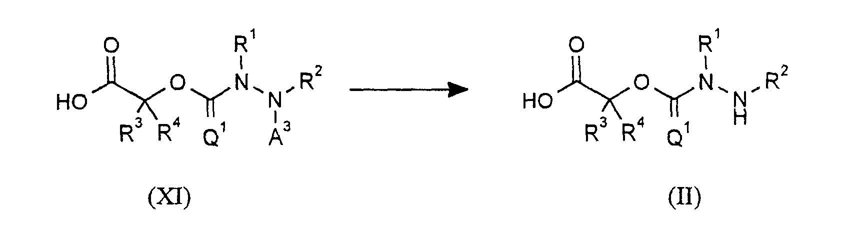

In Formel (X) steht A³ für eine N-terminale Schutzgruppe wie beispielsweise tert.-Butoxycarbonyl (BOC), Benzyloxycarbonyl (Cbz) oder Benzyl (Bzl) (Vgl. z.B. T. W. Greene, P. G. M. Wuts, Protective Groups in Organic Synthesis, 2. Ed., John Wiley & Sons, New York 1991).

Die Reaktion läßt sich mittels üblicher Methoden N-terminaler Deblockierung wie Acidolyse, beispielsweise im Falle der BOC-Gruppe, oder katalytischer Hydrierung, beispielsweise im Falle eines Benzylesters, durchführen.

Die für die Durchführung des Verfahrens (A.a) benötigten O-terminal geschützten Carbazate der Formel (X) oder die Durchführung des Verfahrens (A.b) benötigten N-terminal geschützten Carbazate der Formel (XI) lassen sich herstellen, indem man von N- und O-terminal geschützten Carbazaten der Formel (XII) 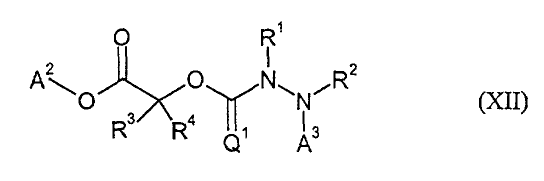 ausgeht und entweder in einem Verfahren (A.a.b) analog zu (A.b) die N-terminale Schutzgruppe abspaltet oder in einem Verfahren (A.b.a) analog zu (A.a) die O-terminale Schutzgruppe abspaltet. Je nach Art der Schutzgruppe kann man in einer besonderen Ausführungsform des Verfahrens auch in einem Schritt beide Schutzgruppen abspalten und von Verbindungen der Formel (XII) direkt zu Verbindungen der Formel (II) gelangen (Verfahren A.a/b).

Verbindungen der Formel (XII) lassen sich z.B. herstellen, indem man Verbindungen der Formel (XIII) mit Carbazaten oder Hydrazinen der Formel (XIV) gegebenenfalls in Gegenwart eines Verdünnungsmittels gemäß dem folgenden Reaktionsschema umsetzt:

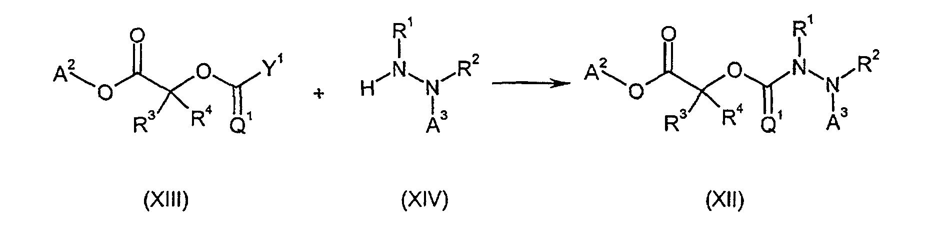

In Formel (XIII) steht Y¹ für Chlor, Trichlormethoxy, C₁-C₄-Alkoxy, gegebenenfalls substituiertes Phenoxy, 1-Imidazolyl oder 1,2,4-Triazolyl.

Carbazate oder Hydrazine der Formel (XIV) sind zum Teil bekannt oder können nach bekannten Methoden erhalten werden (vgl. z.B. J. Chem. Soc. Perkin Trans. I 1975, 1712).

Verbindungen der Formeln (X) und (XII) sind neu, wobei die Verbindungen der Formel (X) ausgenommen sind, in denen R², R⁴ jeweils für Wasserstoff, R¹ für C₁-C₅-Alkyl und R³ für Methyl oder Benzyl stehen, sowie mit Ausnahme der Verbindungen der Formel (X) in denen R¹ für Methyl, R² für Wasserstoff oder Phenylcarbonyl, Q¹ für Sauerstoff, R³ für Phenyl, R⁴ für Wasserstoff und A² für t-Butyl steht, sowie weiterhin mit Ausnahme der Verbindungen, in denen R¹ für Methyl, R² für Phenylcarbonyl, R³ für Methyl, R⁴ für Wasserstoff, Q¹ für Sauerstoff und A² für Benzyl steht, sowie die Verbindungen der Formel (XII) ausgenommen sind, in denen R², R⁴ jeweils für Wasserstoff, R¹ für C₁₋₅-Alkyl und R³ für Methyl oder Benzyl stehen, sowie mit Ausnahme der Verbindungen in denen gleichzeitig R¹ für Methyl, R² für Wasserstoff, R³ für Phenyl, R⁴ für Wasserstoff, Q¹ für Sauerstoff, A² für t-Butyl und A³ für Phenylcarbonyl oder Benzylcarbonyl steht.

Verbindungen der Formel (XIII) lassen sich z.B. herstellen, indem man geschützte α-Hydroxycarbonsäuren der Formel (XV) mit Verbindungen der Formel (VIII) gemäß dem folgenden Reaktionsschema umsetzt:

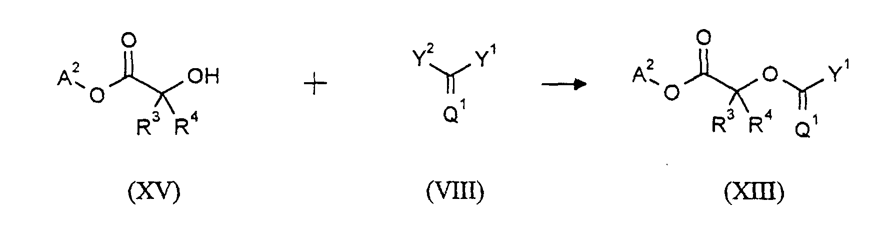

In Formel (VIII) steht Y² für Chlor, Trichlormethoxy, 1-Imidazolyl oder 1,2,4-Triazolyl. Die Verbindungen der Formel (VIII) sind allgemein bekannte (Thio)phosgenierungsreagenzien (vgl. z.B. Org. Syntheses Coll. Vol. **5**, 201 (1973)).

Die geschützten α-Hydroxycarbonsäuren der Formel (XV) lassen sich nach allgemein bekannten Methoden aus den freien Carbonsäuren herstellen (z.B. Veresterung mit Alkyl- oder Benzylhalogeniden in Gegenwart von Caesiumcarbonat, J. Chem. Soc. Perkin Trans. I **1993**, 11). α-Hydroxycarbonsäuren sind käuflich oder lassen sich z.B. aus α-Aminosäuren via Desaminierung herstellen (vgl. z.B. Tetrahedron Letters **28**, 1873 (1987) und **26**, 5257 (1985); Compr. Org. Chem. Vol. 2, 739-778 (1979); Ullmanns Encyclopädie Techn. Chem. 4. Aufl. (1977), Band 13, 163).

Verbindungen der Formel (XII) in der Q¹ für Sauerstoff steht (XII-1), lassen sich z.B. auch herstellen, indem man Verbindungen der Formel (XVI) mit der Formel (XVII) gegebenenfalls in Gegenwart eines Verdünnungsmittels gemäß dem folgenden Reaktionsschema umsetzt:

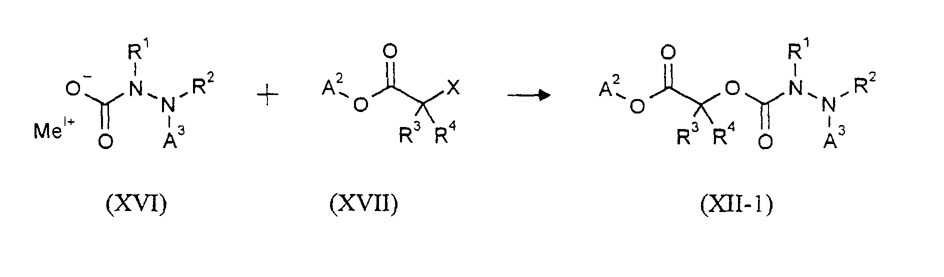

In Formel (XVI) steht Me^{I} für ein Alkalimetall, vorzugsweise Kalium oder Caesium. In Formel (XVII) steht X für Chlor, Brom, Alkansulfonyl, insbesondere Methansulfonyl oder Trifluormethansulfonyl oder Arensulfonyl, insbesondere Benzolsulfonyl oder p-Toluolsulfonyl.

Verbindungen der Formel (XVI) lassen sich herstellen, indem man weiter oben beschriebene Carbazate oder Hydrazine der Formel (XTV) mit Alkalicarbonaten, vorzugsweise Kalium- oder Caesiumcarbonat, gegebenenfalls in Gegenwart von Kohlendioxid umsetzt.

Verbindungen der Formel (XVII) lassen sich z.B. nach allgemein bekannten Methoden aus den o.a. Derivaten von α-Hydroxycarbonsäuren herstellen. Weiterhin kann man Verbindungen der Formel (XVII), in der X für Brom oder Chlor steht z.B. herstellen, indem man zunächst α-Aminosäuren in α-Chlor- oder Bromcarbonsäuren überführt (vgl. J. Am. Chem. Soc. **76**, 6054 (1954); Advances in Protein Chemistry Vol. IV, M.L. Anson, J.T. Edsall (Eds.) **1948**, 33) und diese nach allgemein bekannten Methoden schützt.

Die zur Durchführung der erfindungsgemäßen Verfahrens (B) und (C) benötigten Oxadiazine der Formel (I-c) sind eine Teilmenge der erfindungsgemäßen Verbindungen der allgemeinen Formel (I) und lassen sich beispielsweise nach Verfahren (A) und (D) bis (H) herstellen.

Die weiterhin zur Durchführung des Verfahrens (B) benötigten Verbindungen sind durch die Formel (III) allgemein definiert. In dieser Formel steht R⁴⁻¹ vorzugsweise mit Ausnahme von Wasserstoff für die Reste, die bereits für R⁴ im Zusammenhang mit der Beschreibung der Oxadiazin-Derivate der Formel (I) als bevorzugt genannt wurden. E steht bevorzugt für Halogen, insbesondere Chlor oder Brom, im Falle daß R⁴⁻¹ für einen der gegebenenfalls substituierten Alkylreste steht, auch für Mesyloxy, Tosyloxy oder Trifluormethylsulfonyloxy oder im Falle, daß R⁴⁻¹ für einen Carbonylrest steht, für die Gruppierung -OR⁴⁻¹.

Die Alkylierungs- oder Acylierungsreagenzien der Formel (III) sind allgemein bekannte Reagenzien der organischen Chemie und/oder lassen sich nach bekannten Methoden herstellen.

Die zur Durchführung des erfindungsgemäßen Verfahrens (C) benötigten Aldehyde oder Ketone sind durch die Formel (IV) allgemein definiert. In dieser Formel stehen R⁵ und R⁶ vorzugsweise für diejenigen Reste, die bereits im Zusammenhang mit der Beschreibung der Oxadiazin-Derivate der Formel (I) als bevorzugte Substituenten genannt wurden.

Aldehyde und Ketone der Formel (IV) sind allgemein bekannt und/oder lassen sich nach bekannten Methoden herstellen.

Die zur Durchführung der erfindungsgemäßen Verfahren (D) und (E) benötigten Oxadiazine der Formel (I-e) respektive (I-f) sind Teilmengen der erfindungsgemäßen Verbindungen der allgemeinen Formel (I) und lassen sich beispielsweise nach Verfahren (A) bis (C) und (F) bis (H) herstellen.

Die weiterhin zur Durchführung der Verfahren (D) und (E) benötigten Verbindungen sind durch die Formeln (V) und (VI) allgemein definiert. In diesen Formeln stehen R¹⁻¹ respektive R²⁻¹ vorzugsweise mit Ausnahme von Wasserstoff für die Reste, die bereits für R¹ oder R² im Zusammenhang mit der Beschreibung der Oxadiazin-Derivate der Formel (I) als bevorzugt genannt wurden und E steht sinngemäß bevorzugt für die bei der Beschreibung der Verbindungen der Formel (III) genannten Reste. Sinngemäß bedeutet, daß für Carbonsäureanhydride der Formeln (V) bzw. (VI) die Gruppierungen -OR¹⁻¹ bzw. -OR²⁻¹ an die Stelle von -OR⁴⁻¹ treten.

Die Alkylierungs- oder Acylierungsreagenzien der Formeln (V) und (VI) sind allgemein bekannte Reagenzien der organischen Chemie und/oder lassen sich nach bekannten Methoden herstellen.

Die zur Durchführung des erfindungsgemäßen Verfahrens (F) benötigten Verbindungen sind durch die Formel (VII) allgemein definiert. In dieser Formel stehen R¹, R², R³, R⁴ und Q² vorzugsweise für diejenigen Reste, die bereits im Zusammenhang mit der Beschreibung der Oxadiazin-Derivate der Formel (I) als bevorzugte Substituenten genannt wurden.

Verbindungen der Formel (VII) lassen sich z.B. herstellen, indem man die N-terminale Schutzgruppe A³ von Verbindungen der Formel (XVIII) nach weiter oben angegebenen üblichen Methoden gemäß dem folgenden Reaktionsschema abspaltet: 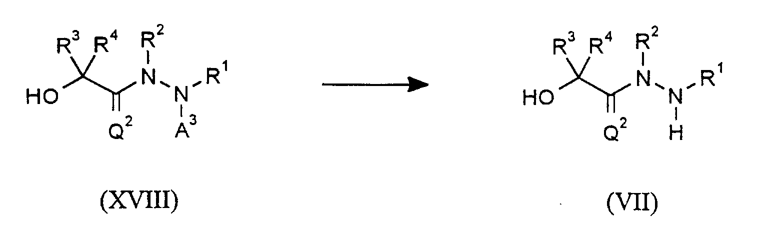 α-Hydroxy(thio)carbonsäurehydrazide der Formel (XVIII) lassen sich z.B. herstellen, indem man α-Hydroxy(thio)carbonsäureester der Formel (XIX) mit Hydrazinen der Formel (XX) gemäß dem folgenden Reaktionsschema umsetzt:

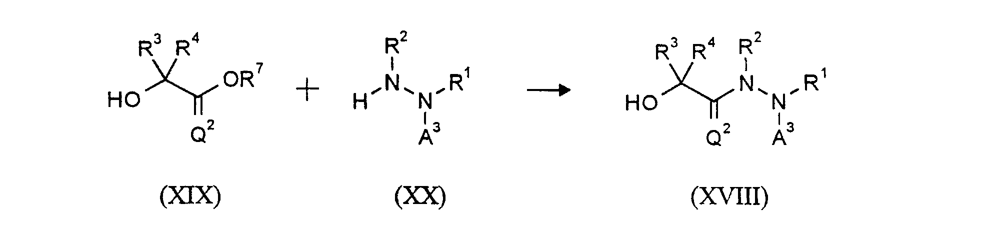

In Formel (XIX) steht R⁷ für gegebenenfalls substituiertes Alkyl oder Aryl. Die α-Hydroxy(thio)carbonsäureester der Formel (XIX) sind käuflich oder lassen sich z.B. aus α-Aminosäuren via Desaminierung herstellen (vgl. z.B. Tetrahedron Letters **28**, 1873 (1987) und **26**, 5257 (1985); Compr. Org. Chem. Vol. 2, 739-778 (1979); Ullmanns Encyclopädie Techn. Chem. 4. Aufl. (1977), Band 13, 163).

Hydrazine der Formel (XX) sind zum Teil bekannt oder können nach bekannten Methoden erhalten werden (vgl. z.B. J. Chem. Soc. Perkin Trans. I **1975**, 1712).

Die zur Durchführung des erfindungsgemäßen Verfahrens (G) benötigten Verbindungen sind durch die Formel (IX) allgemein definiert. In dieser Formel stehen R¹, R², R³, R⁴, Q¹ und Q² vorzugsweise für diejenigen Reste, die bereits im Zusammenhang mit der Beschreibung der Oxadiazin-Derivate der Formel (I) als bevorzugte Substituenten genannt wurden. Y¹ steht bevorzugt für Chlor, Trichlormethoxy, 1-Imidazolyl, 1,2,4-Triazolyl oder Z-substituiertes Aryloxy, insbesondere Pentafluorphenyl, 4-Nitrophenyl oder 2,4-Dinitrophenyl.

Verbindungen der Formel (IX) lassen sich z.B. herstellen, indem man die N-terminale Schutzgruppe A³ von Verbindungen der Formel (XXI) nach weiter oben angegebenen Methoden gemäß dem folgenden Reaktionsschema abspaltet:

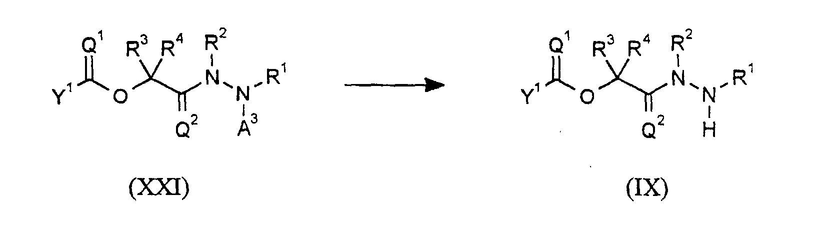

Verbindungen der Formel (XXI) lassen sich z.B. herstellen, indem man oben beschriebene α-Hydroxy(thio)carbonsäurehydrazide der Formel (XVIII) mit weiter oben beschriebenen (Thio)phosgenierungsreagenzien der Formel (VIII) umsetzt und gegebenenfalls das erhaltene Produkt der Formel (XXI), in der Y¹ noch nicht für Z-substituiertes Aryloxy steht, mit einem entsprechenden Phenol oder Phenolat wie beispielsweise 2,4-Dinitrophenol umsetzt.

Die zur Durchführung des erfindungsgemäßen Verfahrens (H) benötigten Oxadiazine der Formel (I-a) sind Teilmengen der erfindungsgemäßen Verbindungen der allgemeinen Formel (I) und lassen sich beispielsweise nach Verfahren (A) bis (G) herstellen.

Die weiterhin zur Durchführung des erfindungsgemäßen Verfahrens (H) benötigten Thionylierungsreagenzien sind vorzugsweise Phosphorpentasulfid oder 2,4-Bis(4-methoxyphenyl)-1,3,2,4-dithiadiphosphetan-2,4-dithion (Lawesson-Reagenz)

Als Reaktionshilfsmittel zur Durchführung des erfindungsgemäßen Verfahrens (A) eignen sich alle Verbindungen die zur Knüpfung einer Amidbindung geeignet sind (vgl. z.B. Houben-Weyl, Methoden der Organischen Chemie, Band 15/2; Bodensky et al.; Peptide Synthesis 2nd ed., Wiley & Sons, New York 1976). Vorzugsweise kommen folgende Methoden in Frage: Aktivestermethode mit Pentafluorphenol (PfP), N-Hydroxysuccinimid, 1-Hydroxybenzotriazol, Kopplung mit Carbodiimiden wie Dicyclohexylcarbodiimid oder N'-(3-Dimethylaminopropyl)-N-ethylcarbodiimid (EBC) sowie die Gemischte-Anhydrid-Methode oder die Kopplung mit Phosphoniumreagenzien wie 1-Benzotriazolyloxy-tris-(dimethylaminophosphonium)-hexafluorphosphat (BOP), Bis-(2-oxo-3-oxazolidinyl)-phosphorylchlorid (BOP-Cl) oder mit Phosphonsäureesterreagenzien wie Cyanphosphonsäurediethylester (DEPC) und Diphenylphosphorylazid (DPPA). Besonders bevorzugt ist die Kopplung mit Bis-(2-oxo-3-oxazolidinyl)-phosphorylchlorid (BOP-C1) und N'-(3-Dimethylaminopropyl)-N-ethylcarbodiimid (EDC) in Gegenwart von 1-Hydroxybenzotriazol (HOBt).

Als Verdünnungsmittel zur Durchführung des erfindungsgemäßen Verfahrens (A) kommen organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-Amylether, Dioxan, Tetrahydrofuran, 1,2-Dimethoxyethan, 1,2-Diethoxyethan, Diethylenglykoldimethylether oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie Formamid, N,N-Dimethylformamid, N,N-Dimethylacetamid, N-Methylformanilid, N-Methylpyrrolidon, 1,3-Dimethyl-tetrahydro-2-pyrimidinon (DMPU), 1,3-Dimethyl-2-imidazolidinon, Tetramethylharnstoff oder Hexamethylphosphorsäuretriamid; N-Oxide wie N-Methylmorpholin-N-oxid; Ester wie Methyl-, Ethyl- oder Butylacetat; Sulfoxide, wie Dimethylsulfoxid; Sulfone, wie Sulfolan.

Die Cyclisierung wird vorzugsweise in Gegenwart einer Base durchgeführt. Als solche kommen anorganische oder organische Basen in Frage. Hierzu gehören vorzugsweise Erdalkalimetall- oder Alkalimetallhydroxide, -alkoholate, -acetate, -carbonate oder -hydrogencarbonate, wie beispielsweise Natrium-, Kalium- oder Ammoniumhydroxid, Natrium-methylat, Natrium-ethylat, Kalium-tert.-butylat, Natrium-, Kalium-, Calcium- oder Ammoniumacetat, Natrium-, Kalium- oder Ammoniumcarbonat, Natriumhydrogen- oder Kaliumhydrogencarbonat sowie tertiäre Amine, wie Trimethylamin, Triethylamin, Tributylamin, Ethyl-diisopropylamin, N,N-Dimethylanilin, N,N-Dimethyl-benzylamin, Pyridin, Picolin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperatur kann bei dem erfindungsgemäßen Verfahren (A) innerhalb eines größeren Bereiches variiert werden. Im allgemeinen arbeitet man bei der Cyclisierung bei Temperaturen zwischen -40°C und +150°C, bevorzugt bei -20°C bis 100°C, besonders bevorzugt bei 0°C bis Raumtemperatur.

Bei der Durchführung des erfindungsgemäßen Verfahrens (A) werden die Verbindung der Formel (II) und die Base im allgemeinen im molaren Verhältnis von 1:1 bis 1:3, vorzugsweise 1:2, eingesetzt.

Das erfindungsgemäße Verfahren (B) kann in Gegenwart eines Verdünnungsmittels durchgeführt werden. Als solches kommen Wasser, organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-Amylether, Dioxan, Tetrahydrofuran, 1,2-Dimethoxyethan, 1,2-Diethoxyethan, Diethylenglykoldimethylether oder Anisol; Amide, wie Formamid, N,N-Dimethylformamid, N,N-Dimethylacetamid, N-Methylformanilid, N-Methylpyrrolidon oder Hexamethylphosphorsäuretriamid; N-Oxide wie N-Methylmorpholin-N-oxid; Ester wie Methyl-, Ethyl- oder Butylacetat; Sulfoxide, wie Dimethylsulfoxid; Sulfone, wie Sulfolan; Alkohole, wie Methanol, Ethanol, n- oder i-Propanol, n-, iso-, sek- oder tert-Butanol, Ethandiol, Propan-1,2-diol, Ethoxyethanol, Methoxyethanol, Diethylenglykolmonomethylether oder Diethylenglykolmonoethylether; Wasser.

Das erfindungsgemäße Verfahren (B) wird vorzugsweise in Gegenwart eines geeigneten Reaktionshilfsmittels durchgeführt. Als solches kommen alle üblichen anorganischen oder organischen Basen in Frage. Hierzu gehören vorzugsweise Erdalkalimetall- oder Alkalimetallhydride, -hydroxide, -alkoholate, -amide, -acetate, -carbonate oder -hydrogencarbonate, wie beispielsweise Natriumhydrid, Natrium-, Kalium- oder Ammoniumhydroxid, Natrium-methylat, Natrium-ethylat, Kalium-tert.-butylat, Natriumamid, Lithiumdiisopropylamid, Bis-(trimethylsilyl)-lithiumamid, -natriumamid, -kaliumamid, Natrium-, Kalium-, Calcium- oder Ammoniumacetat, Natrium-, Kalium- oder Ammoniumcarbonat, Natriumhydrogen- oder Kaliumhydrogencarbonat; Lithiumalkyle wie Methyl-, n-Butyl-, sec.-Butyl oder tert.-Butyllithium sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperatur kann bei dem erfindungsgemäßen Verfahren (B) innerhalb eines größeren Bereiches variiert werden. Im allgemeinen arbeitet man bei Temperaturen zwischen -100°C und +150°C, vorzugsweise zwischen -78°C und 100°C.

Bei der Durchführung des erfindungsgemäßen Verfahrens (B) werden das Oxadiazin-Derivat der Formel (I-c), das Reagenz der Formel (III) und die Base im allgemeinen jeweils in angenähert äquivalenten Mengen verwendet. Es ist jedoch auch möglich, das Reagenz und die Base in einem größeren Überschuß (bis zu 50 Mol) einzusetzen.

Das erfindungsgemäße Verfahren (C) kann in Gegenwart eines Verdünnungsmittels durchgeführt werden. Als solches kommen vorzugsweise die beim Verfahren (B) aufgelisteten in Betracht.

Das erfindungsgemäße Verfahren (C) wird vorzugsweise in Gegenwart eines geeigneten Reaktionshilfsmittels durchgeführt. Als solche kommen alle beim Verfahren (B) aufgelisteten Basen in Frage.

Die Reaktionstemperatur kann bei dem erfindungsgemäßen Verfahren (C) innerhalb eines größeren Bereiches variiert werden. Im allgemeinen arbeitet man bei Temperaturen zwischen -100°C und +120°C, vorzugsweise zwischen -78°C und 100°C.

Zur Eliminierung von Wasser kann, sofern diese nicht selbsttätig stattfindet, eine Säure eingesetzt werden. Als solche kommen alle anorganischen und organischen Protonen- wie auch Lewissäuren, sowie auch alle polymeren Säuren in Frage. Hierzu gehören beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Zinkchlorid, Eisen-III-chlorid, Antimonpentachlorid, saure Ionenaustauscher, saure Tonerden und saures Kieselgel.

Bei der Durchführung des erfindungsgemäßen Verfahrens (C) werden das Oxadiazin-Derivat der Formel (I-c), die Carbonylverbindung der Formel (IV) und die Base im allgemeinen jeweils in angenähert äquivalenten Mengen verwendet. Es ist jedoch auch möglich, das Reagenz und die Base in einem größeren Überschuß (bis zu 50 Mol) einzusetzen.

Die erfindungsgemäßen Verfahren (D) und (E) können in Gegenwart eines Verdünnungsmittels durchgeführt werden. Als solches werden vorzugsweise die beim Verfahren (B) aufgelisteten eingesetzt.

Die erfindungsgemäßen Verfahren (D) und (E) werden vorzugsweise in Gegenwart eines geeigneten Reaktionshilfsmittels durchgeführt. Als solche kommen alle beim Verfahren (B) aufgelisteten Basen in Frage. Darüberhinaus zählen hierzu auch Katalysatoren wie beispielsweise 4-(N,N-Dimethylamino)-pyridin.

Die Reaktionstemperatur kann bei den erfindungsgemäßen Verfahren (D) und (E) innerhalb eines größeren Bereiches variiert werden. Im allgemeinen arbeitet man bei Temperaturen zwischen -40°C und +120°C, vorzugsweise zwischen -10°C und 100°C.

Bei der Durchführung der erfindungsgemäßen Verfahren (D) und (E) werden das Oxadiazin-Derivat der Formel (I-g) bzw. (I-h), die Verbindung der Formel (V) bzw. (VI) und die Base im allgemeinen jeweils in angenähert äquivalenten Mengen verwendet. Es ist jedoch auch möglich, das Reagenz und die Base in einem größeren Überschuß (bis zu 50 Mol) einzusetzen. Vom Katalysator werden gegebenenfalls 0,001 bis 0,1 Mol pro Mol Oxadiazin-Derivat eingesetzt.

Das erfindungsgemäße Verfahren (F) kann in Gegenwart eines Verdünnungsmittels durchgeführt werden. Als solches werden vorzugsweise die beim Verfahren (A) aufgelisteten eingesetzt.

Das erfindungsgemäße Verfahren (F) kann in Gegenwart eines geeigneten Reaktionshilfsmittels durchgeführt werden. Als solches kommen alle beim Verfahren (A) aufgelisteten Basen in Frage.

Die Reaktionstemperatur kann bei dem erfindungsgemäßen Verfahren (F) innerhalb eines größeren Bereiches variiert werden. Im allgemeinen arbeitet man bei Temperaturen zwischen -20°C und +150°C, vorzugsweise zwischen +20°C und 120°C, wobei man gegebenenfalls die Cyclisierung erst nach der Umsetzung der beiden Reaktionspartner durch Temperaturerhöhung einleitet.

Bei der Durchführung des erfindungsgemäßen Verfahrens (F) setzt man pro Mol der Verbindung der Formel (VII) 1,0 bis 2,0 Mol, vorzugsweise 1,0 bis 1,2 Mol Verbindung (VIII) und gegebenenfalls 1,0 bis 5 Mol Reaktionshilfsmittel ein.

Das erfindungsgemäße Verfahren (G) kann in Gegenwart eines Verdünnungsmittels durchgeführt werden. Als solches kommen vorzugsweise die beim Verfahren (A) aufgelisteten in Betracht.

Das erfindungsgemäße Verfahren (G) kann in Gegenwart eines geeigneten Reaktionshilfsmittels durchgeführt werden. Als solches kommen alle beim Verfahren (A) aufgelisteten Basen in Frage.

Die Reaktionstemperatur kann bei dem erfindungsgemäßen Verfahren (G) innerhalb eines größeren Bereiches variiert werden. Im allgemeinen arbeitet man bei Temperaturen zwischen -20°C und +150°C, vorzugsweise zwischen +20°C und 120°C.

Bei der Durchführung des erfindungsgemäßen Verfahrens (G) werden die Verbindung der Formel (II) und die Base im allgemeinen im molaren Verhältnis von 1:1 bis 1:3, vorzugsweise äquimolar eingesetzt.

Das erfindungsgemäße Verfahren (H) kann in Gegenwart eines Verdünnungsmittels durchgeführt werden. Als solche kommen organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-Amylether, Dioxan, Tetrahydrofuran, 1,2-Dimethoxyethan, 1,2-Diethoxyethan, Diethylenglykoldimethylether oder Anisol; Harnstoffe wie 1,3-Dimethyltetrahydro-2-pyrimidinon (DMPU), 1,3-Dimethyl-2-imidazolidinon, Tetramethylharnstoff; Hexamethylphosphorsäuretriamid; Sulfoxide, wie Dimethylsulfoxid; Sulfone, wie Sulfolan.

Die Reaktionstemperatur kann bei dem erfindungsgemäßen Verfahren (H) innerhalb eines größeren Bereiches variiert werden. Im allgemeinen arbeitet man bei Temperaturen zwischen 0°C und +150°C, vorzugsweise zwischen +20°C und 100°C.

Bei der Durchführung des erfindungsgemäßen Verfahrens (H) setzt man pro Mol Verbindung der Formel (I-a) im allgemeinen 1 bis 20, vorzugsweise 1 bis 5 Mol Schwefelungsreagenz ein.

Die Umsetzungen der erfindungsgemäßen Verfahren können bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch Entfernung der flüchtigen Bestandteile gegebenenfalls im Vakuum gereinigt.

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, insbesondere:

Aus der Ordnung der Pseudophyllidea z.B.: Diphyllobothrium spp., Spirometra spp., Schistocephalus spp., Ligula spp., Bothridium spp., Diphlogonoorus spp..

Aus der Ordnung der Cyclophyllidea z.B.: Mesocestoides spp., Anoplocephala spp., Paranoplocephala spp., Moniezia spp., Thysanosmsa spp., Thysaniezia spp., Avitellina spp., Stilesia spp., Cittotaenia spp., Anhyra spp., Bertiella spp., Taenia spp., Echinococcus spp., Hydratigera spp., Davainea spp., Raillietina spp., Hymenolepsis spp., Echinolepsis spp., Echinocotyle spp., Diorchis spp., Dipylidium spp., Joyeuxiella spp., Diplopylidium spp..

Aus der Unterklasse der Monogenea z.B.: Cyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhloccelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonismus spp., Dicrocoelium spp., Collyriclum spp., Nanophyetus spp., Opisthorchis spp., Clonorchis spp., Metorchis spp., Heterophyes spp., Metagonimus spp..

Aus der Ordnung der Enoplida z.B.: Trichuris spp., Capillaria spp., Trichlomosoides spp., Trichinella spp..

Aus der Ordnung des Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostromum spp., Cyclococercus spp., Cylicostephanus spp., Oesophagostomum spp., Chabertia spp., Stephanurus spp., Acylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Gruppe der Gigantohynchida z.B.: Filicollis spp., Moniliformis spp., Macracanthorhynchus spp., Prosthenorchis spp..

Beispielsweise zeigen sie eine hervorragende Wirkung gegen Würmer wie Haemonchus contortus.

Zu den Nutz- und Zuchttieren- gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Zäpfchen, Tabletten, Kapseln, Fasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pouron and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspensionen zur oralen oder dermalen Anwendung sowie zu Injektion; Halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.
Injektionslösungen werden intravenös, intramusculär und subcutan verabreicht.
Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zu Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen, Baden oder Waschen aufgebracht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickugsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß Formulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgieß Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglykole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglycolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfat, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektion angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase gelöst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäure der Kettenlänge C₈₋₁₂ oder andren speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Cypryl/Caprinsäureester von gesättigten Fettalkoholen der Kettelänge C₁₂-C₁₈, Isopropylstearat, Ölsäureoleylester, Ölsäuredecylester, Ethyloleat, Milchsäureethylester, wachsartige Fettsäureester wei künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglykol, Glycerin, Sorbitol und ihre Gemische.
Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker-, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenyl-imidazolthiazol, Benzimidazolcarbamate, Praziquantel, Pyrantel, Febantel.

Anwendungsfertige Zubereitungen enthalten den Wirkstoff in Konzentrationen von 10 ppm bis 20 Gew.-%, bevorzugt von 0,1 bis 10 Gew.-%.

Zubereitungen die vor Anwendung verdünnt werden, enthalten den Wirkstoff in Konzentrationen von 0,5 bis 90 Gew.-%, bevorzugt von 5 bis 50 Gew.-%.

Im allgemeinen hat es sich als vorteilhaft erwiesen, Mengen von etwa 1 bis 100 mg Wirkstoff je kg Körpergewicht pro Tag zur Erzielung wirksamer Ergebnisse zu verabreichen.

Die Wirkstoffe eignen sich weiterhin bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria. Aus der Ordnung der Dermaptera z.B. Forficula auricularia.
Aus der Ordnung der Isoptera z.B. Reticulitermes spp..
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, - Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die erfindungsgemäßen Wirkstoffe zeichnen sich insbesondere durch hervorragende Wirkung gegen die Raupen der Kohlschabe (Plutella maculipennis) aus.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Primiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie
Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus

Hautflügler wie
Sirex juvencus, Urocerus gigas, Urocerus gigas taignus, Urocerus augur

Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze, wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid--Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide, wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on sein.

Die Herstellung und die Verwendung der erfindungsgemäßen Wirkstoffe geht aus den nachfolgenden Beispielen hervor.

### Herstellungsbeispiele

### Beispiel I-1

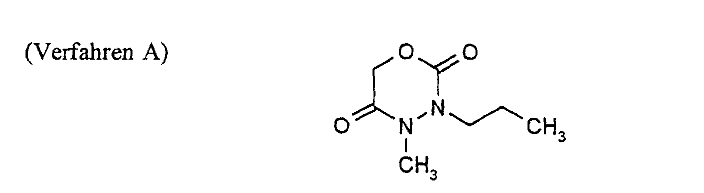

Bei 0°C wurden zu einer Lösung von 3,0 g (2-Methyl-1-propylhydrazyl)-carbonyloxy-essigsäure (z.B. aus Bsp. II-1) in 750 ml trockenem Dichlormethan 5,49 g Ethyldiisopropylamin und anschließend portionsweise 5,19 g Bis-(2-oxo-3-oxazolidinyl)-phosphorylchlorid gegeben. Nach 2 h Rühren bei 0°C wurde über Nacht auf Raumtemperatur erwärmen gelassen. Die Lösung wurde eingeengt und der Rückstand in 300 ml Ethylacetat aufgenommen. Die Lösung wurde mit halbgesättigter NH₄Cl-Lösung und gesättigter NaCl-Lösung gewaschen und mit Natriumsulfat getrocknet. Nach dem Abfiltrieren vom Trockenmittel und Einengen der Lösung wird das Produkt durch Säulenchromatographie gereinigt (stationäre Phase: Kieselgel; mobile Phase Cyclohexan : Ethylacetat = 2 : 1). Man erhielt 1,70 g (71 % der Theorie) 3-Propyl-4-methyl-1,3,4-oxadiazinan-2,5-dion als gelbbraunes Öl.
¹H-NMR (500 MHz, CDCl₃) δ [ppm]: 0,97 (t, 3H, CH₂CH₃), 1,62 (sx, 2H, CH₂CH₃); 3,23 (s, 3H, NCH₃); 3,68 (t, 2H, NH₂); 4,59 (s, 2H, OCH₂)

### Beispiele I-2 bis I-15

Analog zu Beispiel I-1 wurden die in der nachfolgenden Tabelle 1 aufgeführten Verbindungen der Formel (I-j) erhalten.

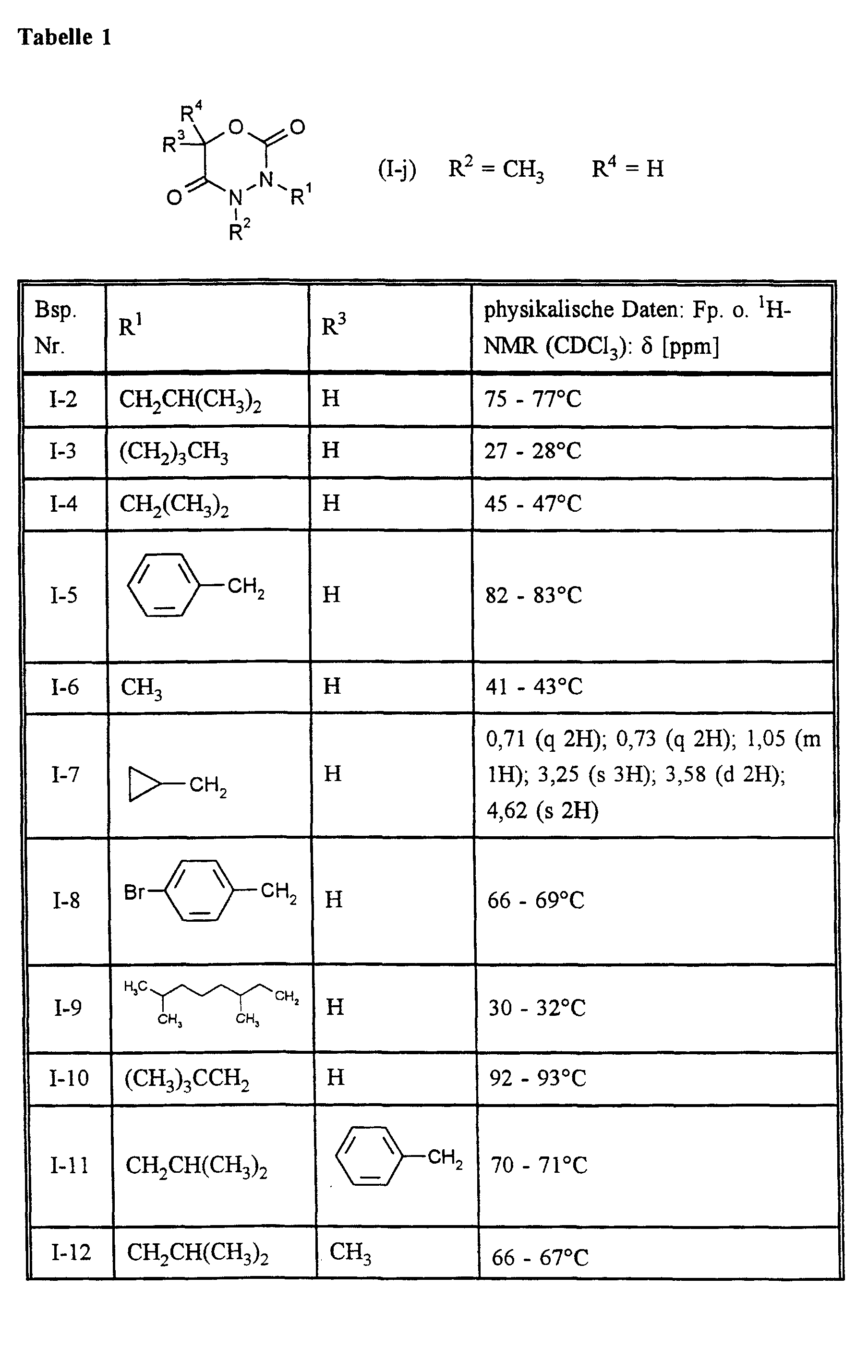

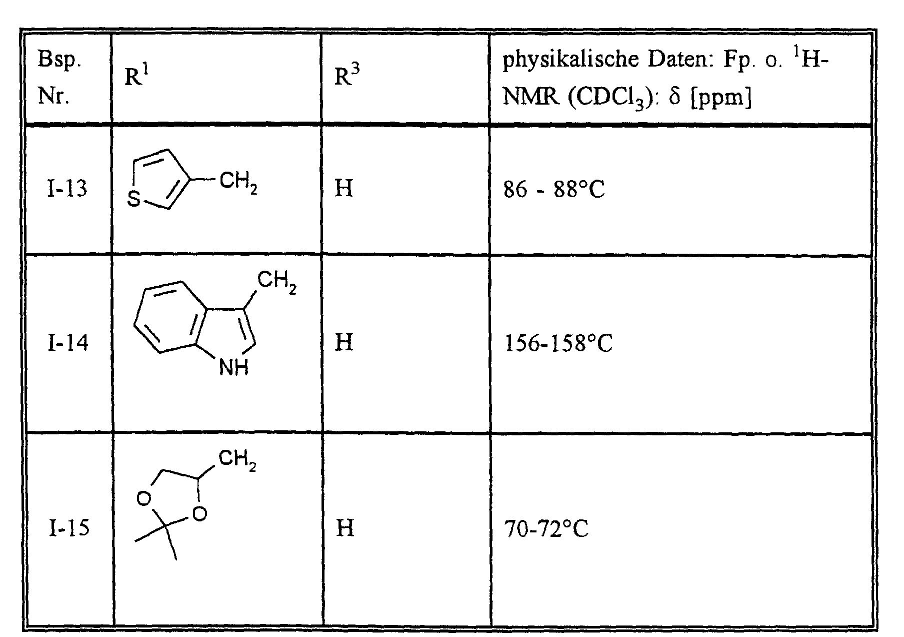

### Beispiel I-16

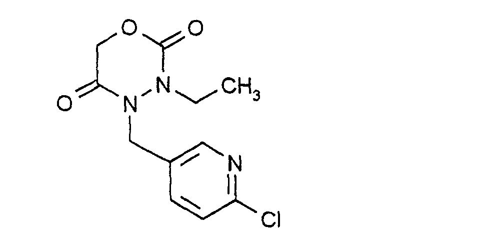

Analog zu Beispiel I-1 wurden aus 5,0 g [2-(2-Chlor-5-pyridyl)-methyl-1-ethylhydrazyl]-carbonyloxy-essigsäure (z.B. aus Beispiel II-16) 3,33 g (79 % d. Th.) 4-(2-Chlor-5-pyridylmethyl)-3-ethyl-1,3,4-oxadiazin-2,5-dion vom Schmelzpunkt 125-128°C erhalten.

### Beispiel II-1

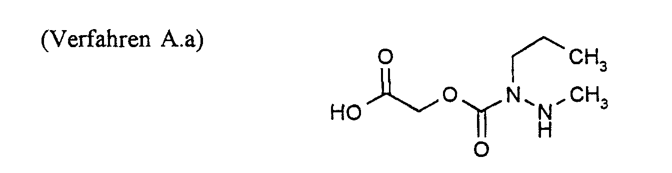

4,36 g (2-Methyl-1-propylhydrazyl)-carbonyloxy-essigsäure-benzylester (z.B. aus Bsp. III-1) wurden in 100 ml Ethylacetat gelöst. Nach Zugabe von 100 mg Pd/C (10 %) als Katalysator wurde die Reaktionslösung bei 1 bar H₂-Druck hydriert. Nach beendeter Reaktion wurde Stickstoff durch die Lösung geleitet und diese anschließend durch Filtration über Kieselgur (Celite®) vom Katalysator befreit. Nach Einengen im Vakuum wurden 3,0 g (100 % der Theorie) (2-Methyl-1-propylhydrazyl)-carbonyloxy-essigsäure als braunes, viskoses Öl erhalten. Das Rohprodukt wurde ohne Reinigung weiter nach Beispiel I-1 umgesetzt.
¹H-NMR (500 MHz, CDCl₃) δ [ppm]: 0,90 (t, 3H, CH₂CH₃); 1,65 (m, 2H CH₂CH₃); 2,65 (br s, 3H; NCH₃); 3,37 (t, 2H, NCH₂); 4,68 (br s, 2H, OCH₂); 6,65 (br s, 2H, NH, CO₂H)

### Beispiele II-2 bis II-12

Analog zu Beispiel II-1 wurden die in der nachfolgenden Tabelle 2 aufgeführten Verbindungen der Formel (II) erhalten.

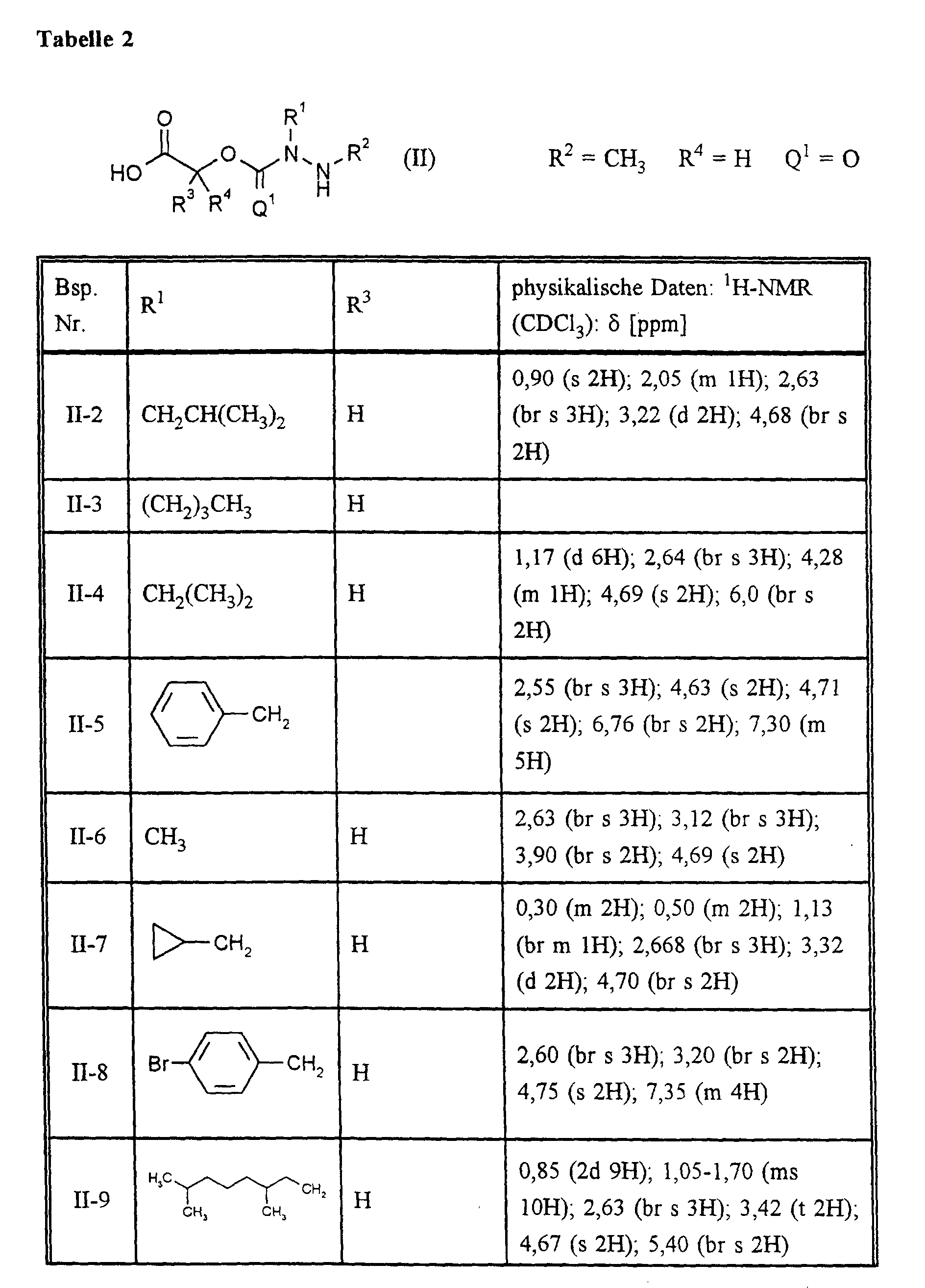

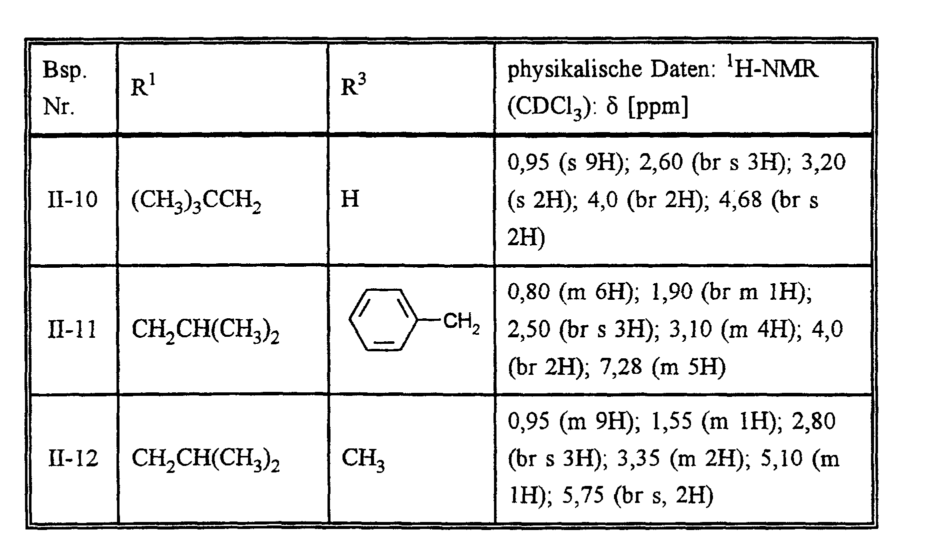

### Beispiel II-13

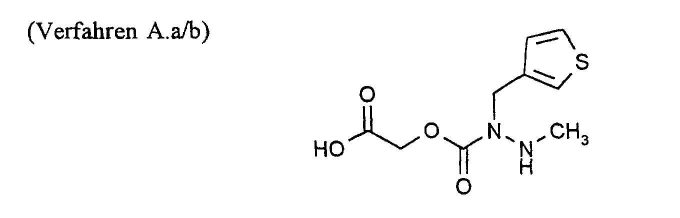

Zu einer Lösung von 10 g [2-tert.-Butoxycarbonyl-2-methyl-1-(3-thienylmethyl)-hydrazyl]-carbonyloxy-essigsäure-tert-butylester (z.B. aus Beispiel IV-13) in 100 ml trockenem Dichlormethan wurden bei 0°C 50,5 g Trifluoressigsäure getropft. Nach 2 h ließ man auf Raumtemperatur erwärmen und kontrollierte die Vollständigkeit der Reaktion per DC (Kieselgel, Cyclohexan : Ethylacetat = 2 : 1). Die Lösung wurde im Vakuum wiederholt unter Zusatz von Dichlormethan zur Verdrängung der Säure eingeengt (zuletzt im Hochvakuum). Es wurden 12,56 g 2-Methyl-1-(3-thienylmethyl)-hydrazyl-carbonyloxy-essigsäure als braunes, viskoses Öl erhalten. Das Rohprodukt wurde ohne Reinigung weiter nach Beispiel I-13 umgesetzt.
¹H-NMR (500 MHz, CDCl₃) δ [ppm]: 2,83 (br s, 3H NCH₃); 4,80 und 4,90 (2s, 2x2H, NCH₂, OCH₂); 7,10 (m, 1H, arom); 7,35 (m, 1H, arom); 7,40 (m, 1H, arom.); 7,63 (br s, 2H, NH, CO₂H)

### Beispiel II-14

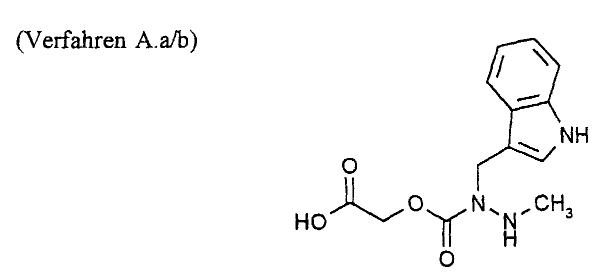

2,5 g [2-Benzyloxycarbonyl-2-methyl-1-(3-indolylmethyl)-hydrazyl]-carbonyloxyessigsäure-benzylester (z.B. aus Bsp. IV-14) wurden in 30 ml Ethanol gelöst. Nach Zugabe von 50 mg Pd/C (10 %) als Katalysator wurde die Reaktionslösung bei 1 bar H₂-Druck hydriert. Nach beendeter Reaktion wurde Stickstoff durch die Lösung geleitet und diese anschließend durch Filtration über Kieselgur (Celite®) vom Katalysator befreit. Nach Einengen im Vakuum wurden 1,3 g (95 % der Theorie) [1-(3-Indolyl)-2-methyl-hydrazyl]-carbonyloxy-essigsäure vom Schmelzpunkt 50 - 53°C erhalten. Das Rohprodukt wurde ohne Reinigung weiter nach Beispiel I-14 umgesetzt.
¹H-NMR (500 MHz, CDCl₃) δ [ppm]: 2,65 (br s, 3H, NCH₃); 4,80 (2s, 4H, OCH₂, NCH₂); 7,0-8,2 (m, 6H, NH, arom)

### Beispiel II-15

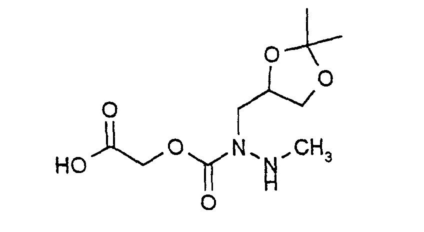

Analog Beispiel II-14 wurde aus [2-Benzyloxycarbonyl-2-methyl-1-(2,2-dimethyl-1,3-dioxolan-4-yl)-methyl-2-methyl-hydrazyl]-carbonyloxy-essigsäure-benzylester (z.B. aus Beispiel IV-15) [1-(2,2-Dimethyl-1,3-dioxolan-4-yl)-methyl-2-methyl-hydrazyl]-carbonyloxy-essigsäure erhalten.
¹H-NMR (500 MHz, CDCl₃) δ [ppm]: 1,35 u. 1,43 (2s, 2x3H, C(CH₃)₂); 2,63 (br s, 3H, NCH₃); 3,60 (br m, 2H, NCH₂); 3,84 (br m, 1H, OCHCH₂); 4,08 u. 4,37 (2 br m, 2x1H, OCH₂CH); 4,68 (br m, 2H, CO-CH₂O); 6,0 (br s, 2H, NH, CO₂H).

### Beispiel II-16

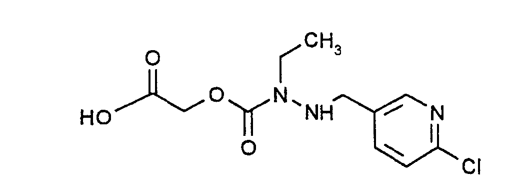

Analog zu Beispiel II-13 wurden aus 7,52 g [2-tert.-Butoxycarbonyl-2-(2-chlor-5-pyridyl)-methyl-1-ethyl-hydrazyl]-carbonyloxy-essigsäure-tert-butylester (z.B. aus Beispiel IV-16) 4,9 g (100 % d. Th.) [2-(2-Chlor-5-pyridyl)-methyl-1-ethylhydrazyl]-carbonyloxy-essigsäure erhalten.
¹H-NMR (500 MHz, CDCl₃) δ [ppm]: 1,10 (br t, 3H, CH₂CH₃); 3,30 (br s, 2H, NCH₂); 4,10 (br s, 2H, NCH₂); 4,72 (s, 2H, OCH₂); 7,43 / 7,89 / 8,38 (3 m, 3 x 1H, arom).

### Beispiel III-1

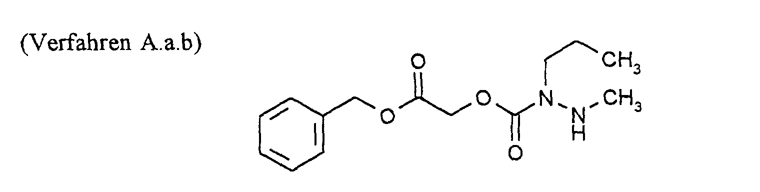

Zu einer Lösung von 6,65 g (2-tert-Butoxycarbonyl-2-methyl-1-propylhydrazyl)-carbonyloxy-essigsäure-benzylester (z.B. aus Bsp. IV-1) in 50 ml trockenem Dichlormethan wurden bei 0°C 16,9 g Trifluoressigsäure zugetropft. Man rührte für 2 bis 3 h bei 0°C und läßt über Nacht auf RT erwärmen. Die Reaktionslösung wurde im Vakuum eingeengt. Der Rückstand wurde in 300 ml Ethylacetat aufgenommen und mit gesättigter Natriumhydrgencarbonat- sowie gesättigter Kochsalz-Lösung gewaschen. Die organische Phase wird über Natriumsulfat getrocknet, vom Trockenmittel abfiltriert und im Vakuum eingeengt. Es wurden 4,76 g (100 % der Theorie) (2-Methyl-1-propylhydrazyl)-carbonyloxy-essigsäure-benzylester als braunes viskoses Öl erhalten. Das Rohprodukt wurde ohne Reinigung weiter nach Beispiel II-1 umgesetzt.
¹H-NMR (500 MHz, CDCl₃) δ [ppm]: 0,88 (t, 3H, CH₂CH₃); 1,64 (sx, 2H, CH₂CH₃); 2,60 (br s, 3H, NCH₃); 3,35 (t, 2H, CH₂CH₃); 4,69 (br s, 2H, CH₂Ph); 5,20 (s, 2H, OCH₂), 7,37 (m, 5H, arom.)

### Beispiele III-2 bis III-12

Analog zu Beispiel III-1 wurden die in der nachfolgenden Tabelle 3 aufgeführten Verbindungen der Formel (X) erhalten.

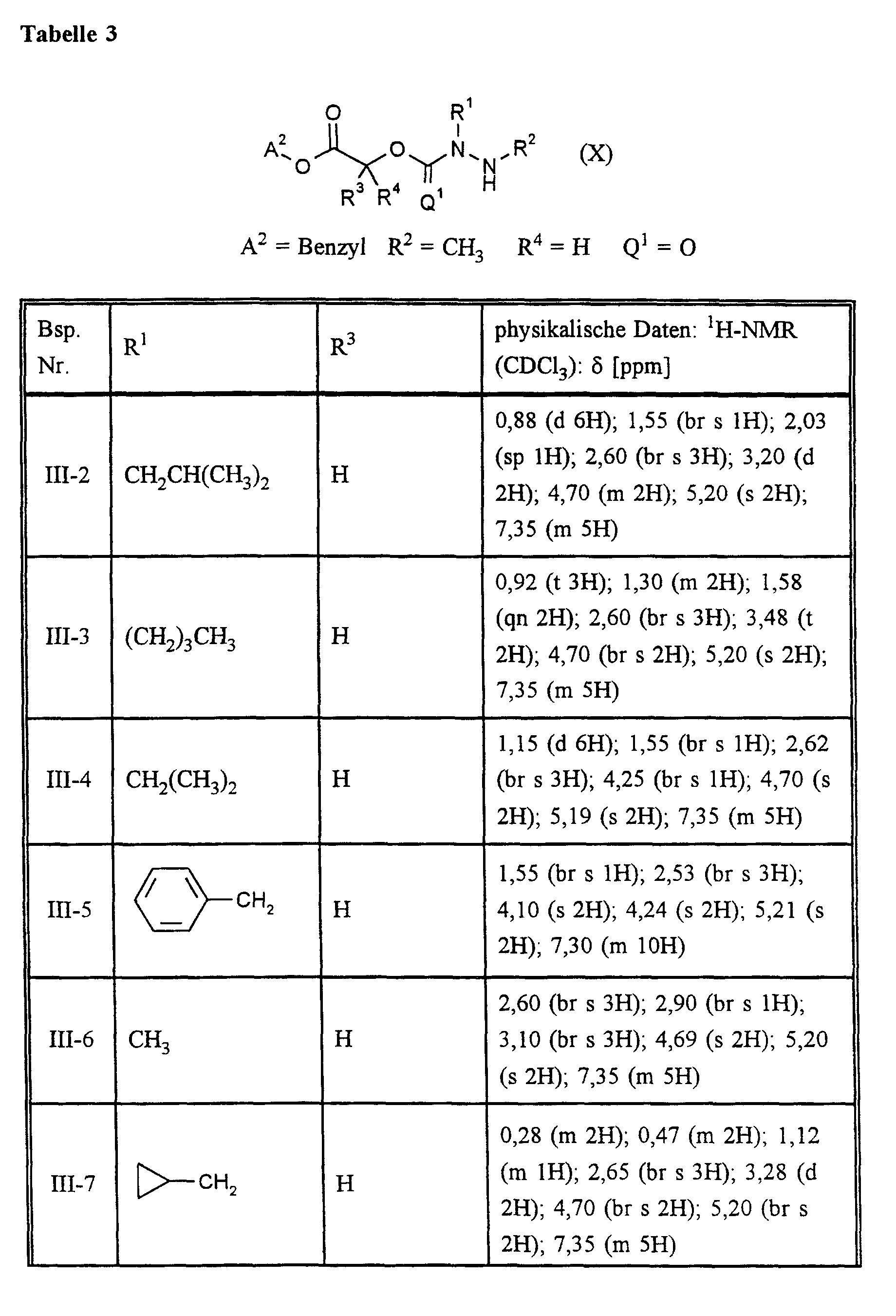

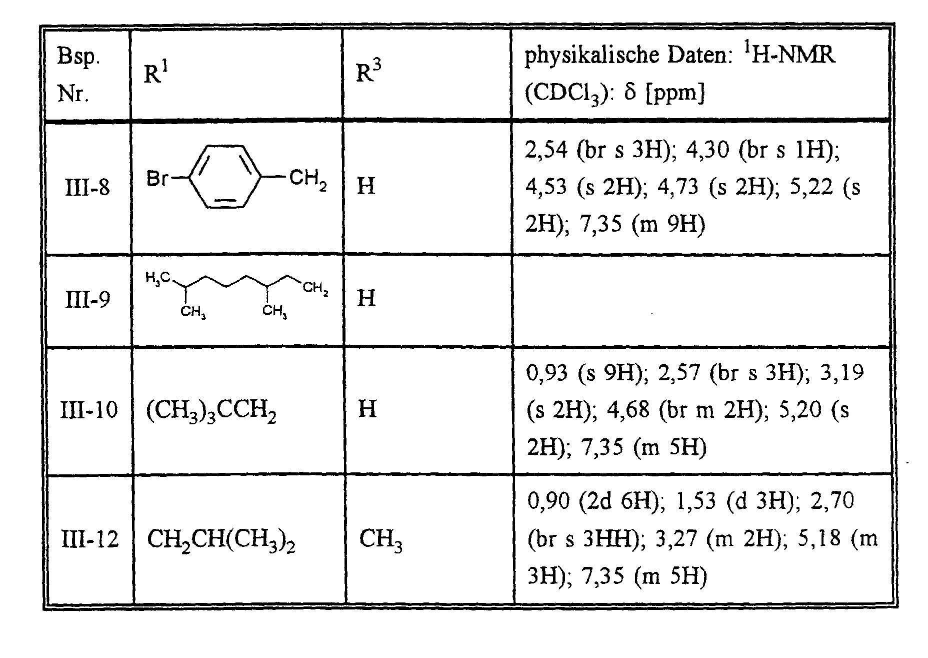

### Beispiel IV-1

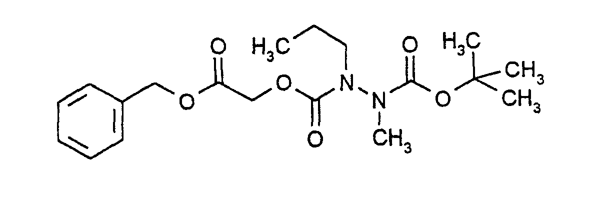

In eine Suspension aus 22,80 g Caesiumcarbonat und 6,55 g 1-tert.-Butoxycarbonyl-1-methyl-2-propyl-hydrazin in 140 ml trockenem Dimethylformamid wurde für ca. 1 h Kohlendioxid-Gas eingeleitet. Darauf wurden 8,02 g Bromessigsäurebenzylester langsam zugetropft und für weitere 30-45 min. Kohlendioxid eingeleitet. Nach Rühren über Nacht wurde die Reaktionsmischung auf 300 ml halbgesättigte Kochsalz-Lösung gegeben und mit 3mal 150 ml Ethylacetat extrahiert. Die organische Phase wurde über Natriumsulfat getrocknet, vom Trockenmittel abfiltriert und im Vakuum bei bis zu 50°C eingeengt. Das Produkt wurde durch Säulenchromatographie gereinigt (Kieselgel; Cyclohexan : Ethylacetat = 5 : 1). Es wurden 8,9 g (67 % der Theorie) (2-tert.-Butoxycarbonyl-2-methyl-1-propylhydrazyl)-carbonyloxy-essigsäure-benzylester als hellgelbes viskoses Öl erhalten.
¹H-NMR (500 MHz, CDCl₃) δ [ppm]: 0,93 (t, 3H, CH₂CH₃); 1,47 (ms, 9H, C(CH₃)₃; 1,63 (m, 2H, CH₂CH₃); 2,90-3,55 (ms, 5H, NCH₂, NCH₃); 4,40-5,25 (ms, 4H, CH₂Ph, OCH₂); 7,36 (m, 5H, C₆H₅)

### Beispiel IV-2

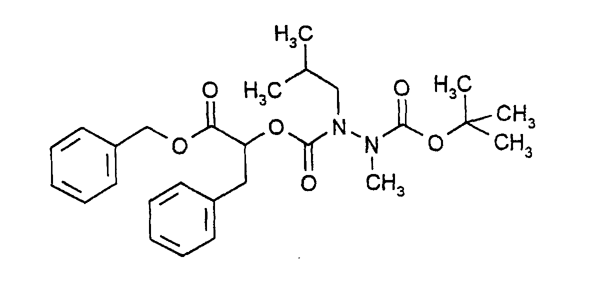

Zu einer Lösung von 19,8 g Phosgen in 100 ml Toluol wurden bei 0-5°C über 1 h eine Lösung von 25,63 g D-Phenylmilchsäurebenzylester und 11,13 g Triethylamin in 130 ml trockenem Tetrahydrofuran getropft. Es wurden ca. 30 min. bei 0°C und 1 h bei Raumtemperatur nachgerührt. Die Lösung wurde vom ausgefallenen Feststoff abfiltriert und im Vakuum eingeengt. Der erhaltene rohe Chlorameisensäureester wurde in 50 ml trockenem Tetrahydrofuran aufgenommen und über 1-2 h zu einer auf 0°C gekühlten Lösung von 20,24 g 1-tert.-Butoxycarbonyl-1-methyl-2-sec.-butyl-hydrazin und 10,12 g Triethylamin in 70 ml trockenem Tetrahydrofuran getropft. Anschließend wurde auf Raumtemperatur erwärmen gelassen und über Nacht gerührt. Die Reaktionslösung wurde auf 200 ml halbgesättigte Kochsalz-Lösung gegeben und mit 3mal 150 ml Ethylacetat extrahiert. Die organische Phase wurde über Natriumsulfat getrocknet, vom Trockenmittel abfiltriert und im Vakuum eingeengt. Das Produkt wurde durch Säulenchromatographie gereinigt (Kieselgel; Cyclohexan : Ethylacetat = 10 : 1). Es wurden 36,83 g (76 % der Theorie) 1-(2-tert.-Butoxycarbonyl-2-methyl-1-sec.-butylhydrazyl)-carbonyloxy-2-phenylpropionsäure-benzylester als hellgelbes viskoses Öl erhalten.
¹H-NMR (500 MHz, CDCl₃) δ [ppm]: 0,90 (ms, 6H, CH(CH₃)₂); 1,30-1,75 (ms, 10H, CH(CH₃)₂, C(CH₃)₃); 2,60-3,40 (ms, 6H, OCH, NCH₂, NCH₃); 5,0-5,40 (ms, 4H, 2 CH₂Ph); 7,25 (m, 10H, 2 C₆H₅)

### Beispiele IV-3 bis IV-12

Analog zu den Beispielen IV-1 und IV-2 wurden die in der nachfolgenden Tabelle 4 aufgeführten Verbindungen der Formel (XII) erhalten.

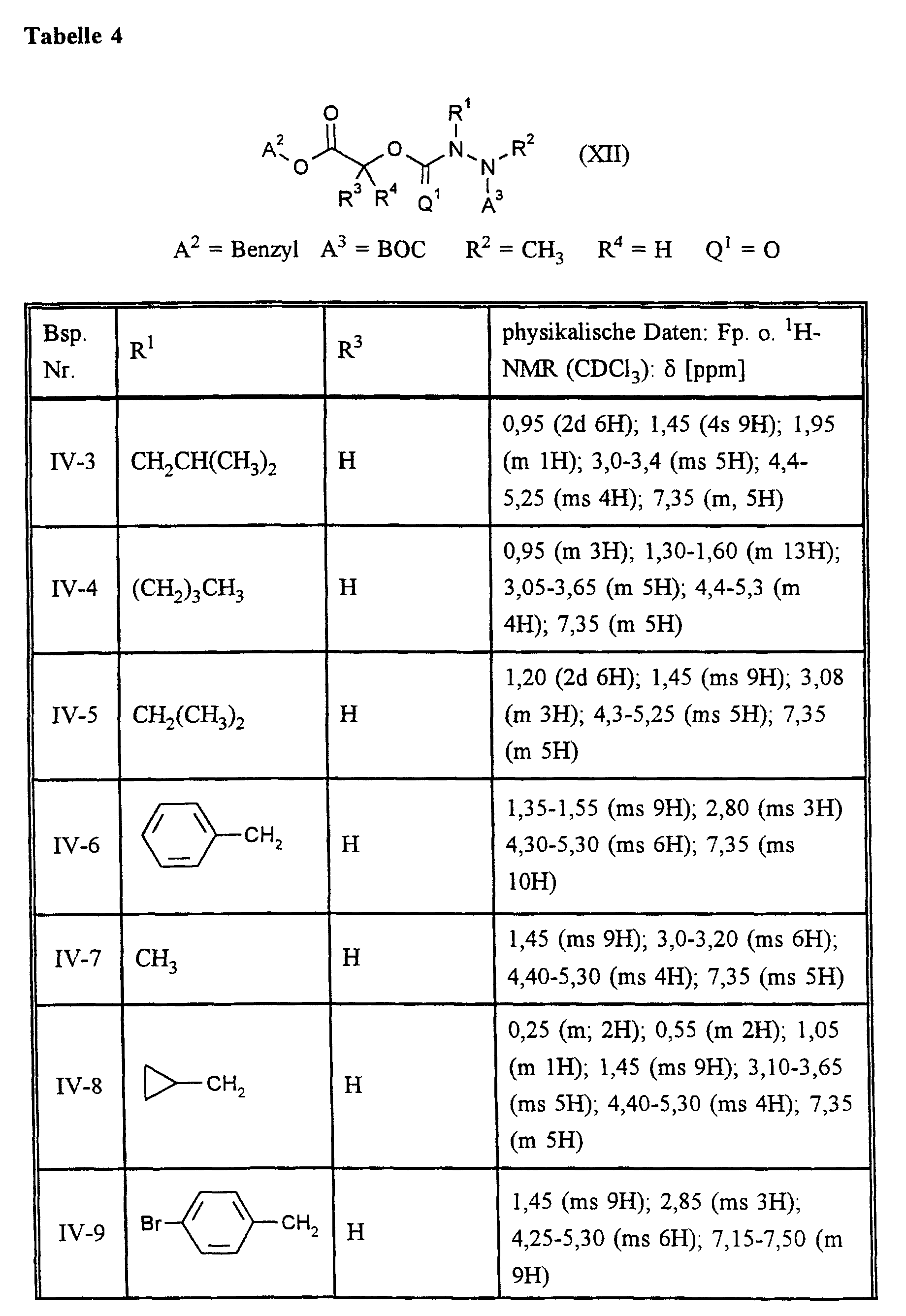

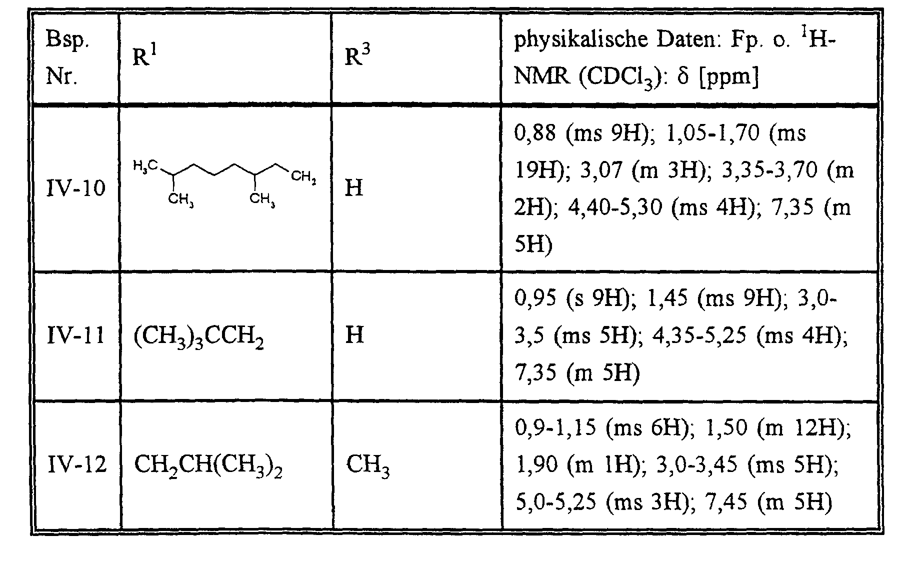

### Beispiel IV-13

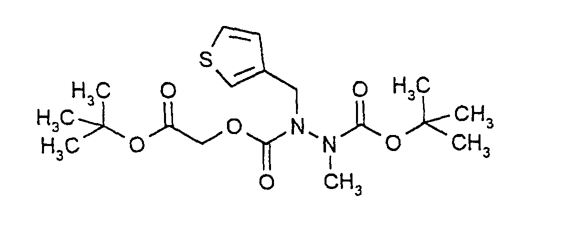

In eine Suspension aus 22,80 g Caesiumcarbonat in 140 ml trockenem Dimethylformamid wurde für ca. 20 min. Kohlendioxid-Gas eingeleitet. Darauf wurden 8,47 g 1-tert-Butoxycarbonyl-1-methyl-2-(3-thienylmethyl)-hydrazin langsam hinzugetropft. Es wurde weitere 60 min. Kohlendioxid eingeleitet, anschließend 6,83 g Bromessigsäure-tert.-butylester langsam zugetropft und für weiter 30-45 min. Kohlendioxid eingeleitet. Nach Rühren über Nacht wurde die Reaktionsmischung auf 200 ml halbgesättigte Kochsalz-Lösung gegeben und mit 3mal 150 ml Ethylacetat extrahiert. Die organische Phase wurde über Natriumsulfat getrocknet, vom Trockenmittel abfiltriert und im Vakuum bei bis zu 50°C eingeengt. Das Produkt wurde durch Säulenchromatographie gereinigt (Kieselgel; Cyclohexan : Ethylacetat = 5 : 1). Es wurden 14,1 g (100 % der Theorie) [2-tert.-Butoxycarbonyl-2-methyl-1-(3-thienylmethyl)-hydrazyl]-carbonyloxy-essigsäure-tert.-butylester erhalten.
¹H-NMR (500 MHz, CDCl₃) δ [ppm]: 1,35-1,55 (m, 18H, 2x C(CH₃)₃); 2,87 (m, 3H, NCH₃); 4,25-5,05 (ms, 4H, OCH₂, NCH₂); 7,20 (m, 3H, arom.)

### Beispiel IV-14

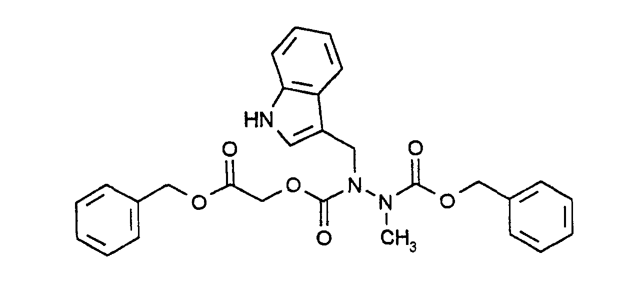

Analog Beispiel IV-1 wurden aus 10,8 g 1-Benzyloxycarbonyl-1-methyl-2-(3-indolylmethyl)-hydrazin und 8,02 g Bromessigsäurebenzylester in drei Fraktionen 6,41 g reiner und 7,04 g leicht verunreinigter (76 % der Theorie) [2-Benzyloxycarbonyl-2-methyl-1-(3-indolylmethyl)-hydrazyl]-carbonyloxy-essigsäure-benzylester erhalten.
¹H-NMR (500 MHz, CDCl₃) δ [ppm]: 2,83 (m, 3H, NCH₃); 4-10-5,35 (ms 6H, NH₂, OCH₂, CH₂Ph); 7,35 (m, 15H, arom); 8,05 (br s, 1H, NH)

### Beispiel IV-15

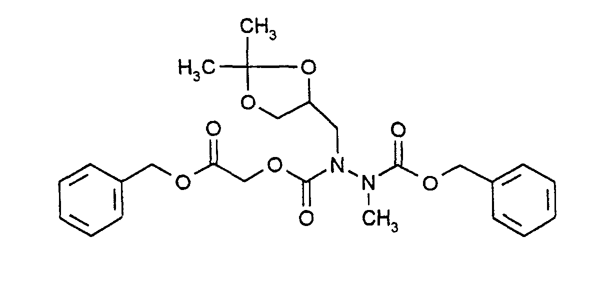

Analog Beispiel IV-1 wurde aus 1-Benzyloxycarbonyl-1-methyl-2-(2,2-dimethyl-1,3-dioxolan-4-yl)-methyl-hydrazin und Bromessigsäurebenzylester [2-Benzyloxycarbonyl-2-methyl-1-(2,2-dimethyl-1,3-dioxolan-4-yl)-methyl-2-methyl-hydrazyl]-carbonyloxy-essigsäure-benzylester erhalten.
¹H-NMR (500 MHz, CDCl₃) δ [ppm]: 1,23 bis 1,43 (m, 6H, C(CH₃)₂; 3,12-3,20 (m, 3H, NCH₃); 3,25-4,97 (ms, 7H, OCH₂, NCH₂, NCH₂CH, OCHCH₂O); 5,17 (m, 4H, 2xCH₂Ph); 7,35 (m, 1H, arom).

### Beispiel IV-16

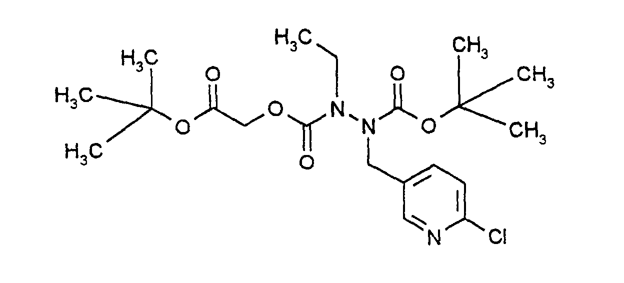

Analog zu Beispiel IV-13 wurden aus 7,13 g 1-tert.-Butoxycarbonyl-1-(2-chlor-5-pyridyl)-methyl-2-ethyl-hydrazin und 4,87 g Bromessigsäure-tert-butylester, 955 g (86 % d.Th.) [2-tert.-Butoxycarbonyl-2-(2-Chlor-5-pyridyl)-methyl-1-ethylhydrazyl]-carbonyloxy-essigsäure-tert.-butylester erhalten.
¹H-NMR (500 MHz, CDCl₃): 1,0 (m, 3H, CH₃CH₂); 1,47 (ms, 18H, 2 x C(CH₃)₃); 3,15-3,45 (ms, 2H, NCH₂); 4,05-5,0 (ms, 4H, NCH₂,OCH₂); 7,30 / 7,75 / 8,40 (3m, 3 x 1H, arom).

### Anwendungsbeispiele

### Beispiel A

### Haemonchus contortus / Schaf

Experimentell mit Haemonchus contortus infizierte Schafe wurden nach Ablauf der Präpatenzzeit des Parasiten behandelt.
Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Dabei wurden die folgenden Ergebnisse erhalten:

| Wirkstoff Beispiel Nr. | effektive Dosis in mg/kg |
|---|---|
| I-2 | 5 |
| I-3 | 5 |
| I-11 | 5 |

### Beispiel B

### Plutella-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigte z.B. die Verbindung gemäß Herstellungsbeispiel I-2 bei einer beispielhafte Wirkstoffkonzentration von 0,1 % nach 7 Tagen einen Abtötungsgrad von 100 %.

## Patentansprüche

1. Oxadiazin-Derivate der Formel (I) 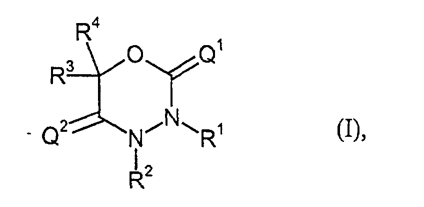 in welcher
R¹ und R² unabhängig voneinander für Wasserstoff, jeweils gegebenenfalls durch Halogen substituiertes Alkyl, Hydroxyalkyl, Alkanoyloxyalkyl, Alkoxyalkyl, Arylalkoxyalkyl, Mercaptoalkyl, Alkylthioalkyl, Alkylsulfinylalkyl, Alkylsulfonylalkyl, Carboxyalkyl, Alkoxycarbonylalkyl, Aryloxycarbonylalkyl, Arylalkyloxycarbonylalkyl, Carbamoylalkyl, Aminoalkyl, Alkylaminoalkyl, Dialkylaminoalkyl, Alkoxycarbonylaminoalkyl, Alkylcarbonyl, Cycloalkylcarbonyl oder für jeweils gegebenenfalls substituiertes Cycloalkyl, Cycloalkylalkyl, Aryl, Arylalkyl, Arylcarbonyl, Heterocyclylalkyl, Hetaryl oder Hetarylalkyl stehen oder
R¹, R² und die beiden Stickstoffatome an die sie gebunden sind für einen gegebenenfalls substituierten heterocyclischen Ring stehen,
R³ und R⁴ unabhängig voneinander für Wasserstoff, jeweils gegebenenfalls durch Halogen substituiertes Alkyl, Alkenyl, Hydroxyalkyl, Alkanoyloxyalkyl, Alkoxyalkyl, Arylalkoxyalkyl, Mercaptoalkyl, Alkylthioalkyl, Alkylsulfinylalkyl, Alkylsulfonylalkyl, Alkoxycarbonylalkyl, Aryloxycarbonylalkyl, Arylalkyloxycarbonylalkyl, Carbamoylalkyl, Aminoalkyl, Alkylaminoalkyl, Dialkylaminoalkyl, Alkylcarbonyl, Cycloalkylcarbonyl oder für jeweils gegebenenfalls substituiertes Cycloalkyl, Cycloalkylalkyl, Aryl, Arylalkyl, Hetaryl oder Hetarylalkyl stehen oder
R³ und R⁴ gemeinsam für Alkylen oder den Rest (a) 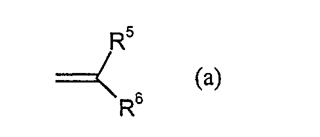 stehen, worin
R⁵ und R⁶ unabhängig voneinander für Wasserstoff, jeweils gegebenenfalls durch Halogen substituiertes Alkyl, Alkenyl, Hydroxyalkyl, Alkanoyloxyalkyl, Alkoxyalkyl, Arylalkoxyalkyl, Mercaptoalkyl, Alkylthioalkyl, Alkylsulfinylalkyl, Alkylsulfonylalkyl, Alkoxycarbonylalkyl, Aryloxycarbonylalkyl, Aminoalkyl, Alkylaminoalkyl, Dialkylaminoalkyl, Alkylcarbonyl oder für jeweils gegebenenfalls substituiertes Cycloalkyl, Cycloalkylalkyl, Aryl, Arylalkyl, Hetaryl oder Hetarylalkyl stehen und
Q¹ und Q² unabhängig voneinander für Sauerstoff oder Schwefel stehen,
wobei 6,6-Diphenyl-(1,3,4)-oxadiazinan-2,5-dion ausgenommen ist.

2. Verfahren zur Herstellung der Oxadiazin-Derivate der Formel (I) gemäß Anspruch 1 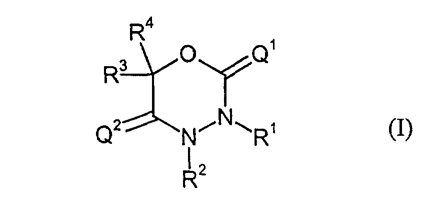 in welcher
R¹ bis R⁴, Q¹ und Q² die in Anspruch 1 genannte Bedeutung haben, **dadurch gekennzeichnet, dass** man
A) für den Fall, dass (1,3,4)-Oxadiazin-Derivate der Formel (I-a) 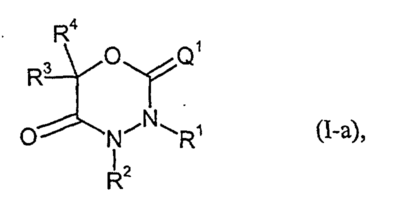 in welcher
R¹ bis R⁴ und Q¹ die in Anspruch 1 angegebenen Bedeutungen haben,
hergestellt werden, Carbazate der Formel (II) 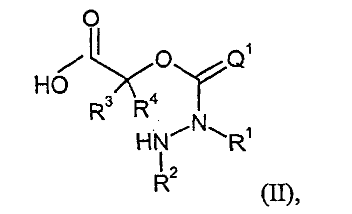 in welcher
R¹ bis R⁴ und Q¹ die in Anspruch 1 angegebenen Bedeutungen haben,
in Gegenwart eines Reaktionshilfsmittels und eines Verdünnungsmittels und gegebenenfalls in Gegenwart einer Base umsetzt,
B) für den Fall, dass (1,3,4)-Oxadiazin-Derivate der Formel (I-b) 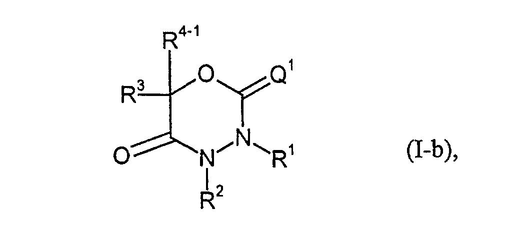 in welcher
R¹ bis R³, Q¹ und Q² die in Anspruch 1 angegebenen Bedeutungen haben und
R⁴⁻¹ mit Ausnahme von Wasserstoff für dieselben Reste wie die für R⁴ in Anspruch 1 angegeben steht,
hergestellt werden,
(1,3,4)-Oxadiazin-Derivate der Formel (I-c) 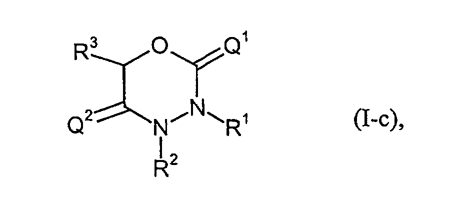 in welcher
R¹ und R² die in Anspruch 1 angegebenen Bedeutungen außer Wasserstoff haben,
R³, Q¹ und Q² die in Anspruch 1 angegebenen Bedeutungen haben
mit Verbindungen der Formel (III)
R⁴⁻¹-E (III),
in welcher
R⁴⁻¹ die in Anspruch 1 angegebene Bedeutung hat und
E für eine elektronenziehende Abgangsgruppe steht,
gegebenenfalls in Gegenwart eines Verdünnungsmittels und gegebenenfalls in Gegenwart eines Reaktionshilfsmittels umsetzt,
C) für den Fall, dass (1,3,4)-Oxadiazin-Derivate der Formel (I-d) 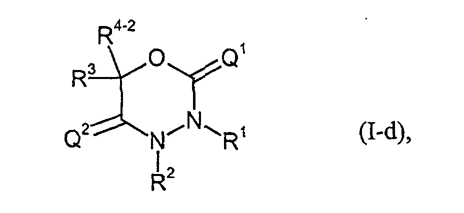 in welcher
R¹ bis R³, Q¹ und Q² die in Anspruch 1 angegebenen Bedeutungen haben und
R⁴⁻² für den Rest (b) 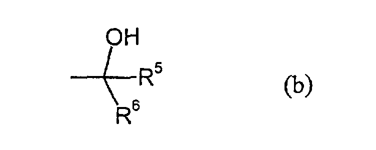 steht, worin
R5 und R6 unabhängig voneinander für Wasserstoff, gegebenenfalls substituiertes Alkyl oder Aryl steht, oder
R³ und R⁴⁻² gemeinsam für den Rest (a) 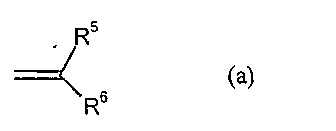 stehen, worin
R⁵ und R⁶ die in Anspruch 1 angegebenen Bedeutungen haben,
hergestellt werden,
(1,3,4)-Oxadiazin-Derivate der Formel (I-c) 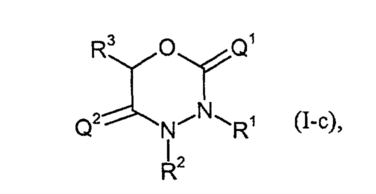 in welcher
R¹ bis R³, Q¹ und Q² die in Anspruch 1 angegebenen Bedeutungen haben
mit Ketonen oder Aldehyden der Formel (IV)
R⁵-CO-R⁶ (IV),
in welcher
R⁵ und R⁶ die in Anspruch 1 angegebenen Bedeutungen haben,
gegebenenfalls in Gegenwart eines Verdünnungsmittels und gegebenenfalls in Gegenwart eines Reaktionshilfsmittels umsetzt und gegebenenfalls anschließend Wasser eliminiert,
D) für den Fall, dass (1,3,4)-Oxadiazin-Derivate der Formel (I-e) 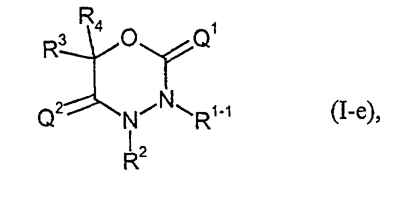 in welcher
R¹⁻¹ mit Ausnahme von Wasserstoff für dieselben Reste wie die für R¹ in Anspruch 1 angegebenen steht,
R² bis R⁴, Q¹ und Q² die in Anspruch 1 angegebenen Bedeutungen haben,
hergestellt werden, (1,3,4)-Oxadiazin-Derivate der Formel (I-f) 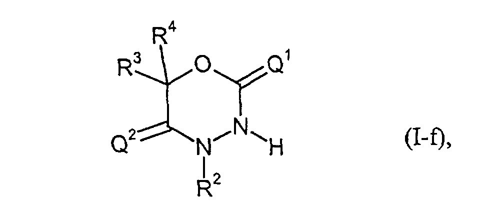 in welcher
R² bis R⁴, Q¹ und Q² die in Anspruch 1 angegebenen Bedeutungen haben
mit Verbindungen der Formel (V)
R¹⁻¹-E (V),
in welcher
R¹⁻¹ die oben angegebene Bedeutung hat und
E für eine elektronenziehende Abgangsgruppe steht,
gegebenenfalls in Gegenwart eines Verdünnungsmittels und gegebenenfalls in Gegenwart eines Reaktionshilfsmittels umsetzt,
E) für den Fall, dass (1,3,4)-Oxadiazin-Derivate der Formel (I-g) 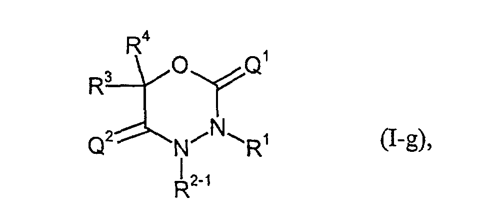 in welcher
R¹, R³, R⁴, Q¹ und Q² die in Anspruch 1 angegebenen Bedeutungen haben und
R²⁻¹ mit Ausnahme von Wasserstoff für dieselben Reste wie die für R² in Anspruch 1 angegebenen steht,
hergestellt werden, (1,3,4)-Oxadiazin-Derivate der Formel (I-h) 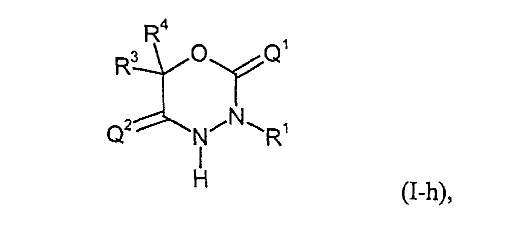 in welcher
R¹, R³, R⁴, Q¹ und Q² die in Anspruch 1 angegebenen Bedeutungen haben
mit Verbindungen der Formel (VI)
R²⁻¹-E (VI),
in welcher
R²⁻¹ die oben angegebene Bedeutung hat und
E für eine elektronenziehende Abgangsgruppe steht,
gegebenenfalls in Gegenwart eines Verdünnungsmittels und gegebenenfalls in Gegenwart eines Reaktionshilfsmittels umsetzt,
F) für den Fall, dass (1,3,4)-Oxadiazin-Derivate der Formel (I) 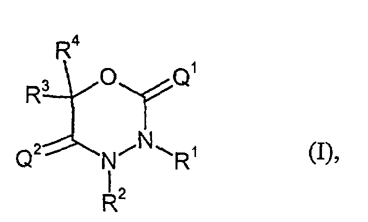 in welcher
R¹ bis R⁴, Q¹ und Q² die in Anspruch 1 angegebenen Bedeutungen haben, wobei 6,6-Diphenyl-(1,3,4)-oxadiazinan-2,5-dion ausgenommen ist,
hergestellt werden, Verbindungen der Formel (VII) 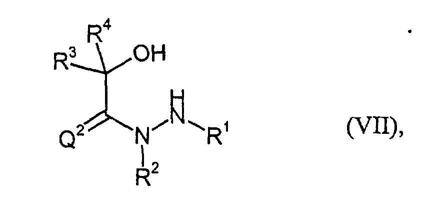 in welcher
R¹ bis R⁴ und Q² die in Anspruch 1 angegebenen Bedeutungen haben,
mit Verbindungen der Formel (VIII) 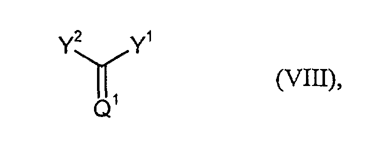 in welcher
Y1 für Chlor, Trichlormethoxy, C₁-C₄-Alkoxy, gegebenenfalls substituiertes Phenoxy, 1-Imidazolyl oder 1,2,4-Triazolyl steht und
Y² für Chlor, Trichlormethoxy, 1-Imidazolyl oder 1,2,4-Triazolyl steht,
Q¹ die in Anspruch 1 angegebene Bedeutung hat,
gegebenenfalls in Gegenwart eines Verdünnungsmittels und gegebenenfalls in Gegenwart eines Reaktionshilfsmittels umsetzt und cyclokondensiert,
G) für den Fall, dass (1,3,4)-Oxadiazin-Derivate der Formel (I) 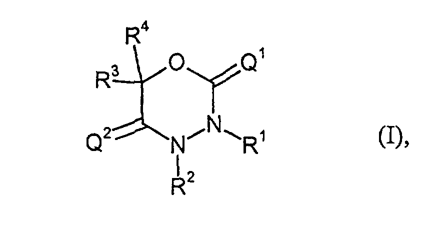 in welcher
R¹ bis R⁴, Q¹ und Q² die in Anspruch 1 angegebenen Bedeutungen haben,
hergestellt werden, Verbindungen der Formel (IX) 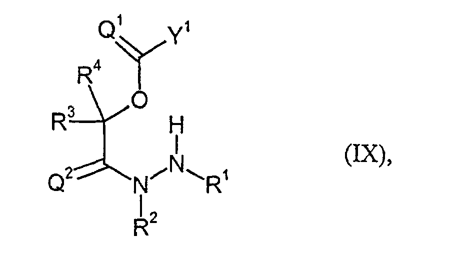 in welcher
R¹ bis R⁴, Q¹, Q² und Y¹ die in Anspruch 1 angegebenen Bedeutungen haben und
gegebenenfalls in Gegenwart eines Verdünnungsmittels und gegebenenfalls in Gegenwart eines Reaktionshilfsmittels cyclokondensiert,
H) für den Fall, dass (1,3,4)-Oxadiazin-Derivate der Formel (I-i) 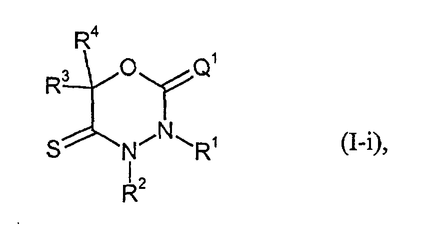 in welcher
R¹ bis R⁴ und Q¹ die in Anspruch 1 angegebenen Bedeutungen haben,
hergestellt werden, (1,3,4)-Oxadiazin-Derivate der Formel (I-a) 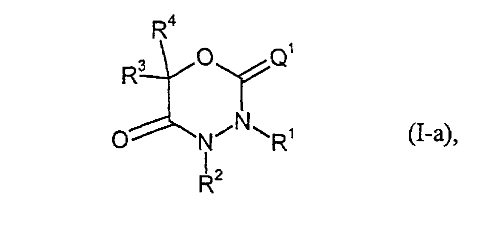 in welcher
R¹ bis R⁴ und Q die in Anspruch 1 angegebenen Bedeutungen haben,
mit einem Thionylierungsreagenz gegebenenfalls in Gegenwart eines Verdünnungsmittels umsetzt.

3. Carbazate der Formel (II) 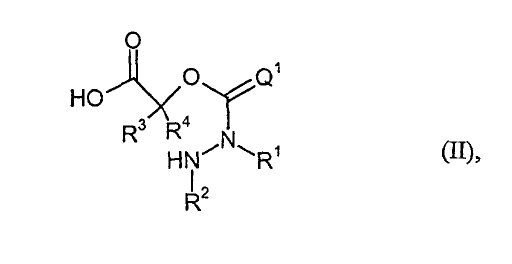 in welcher
R¹, R², R³, R⁴ und Q¹ die in Anspruch 1 angegebenen Bedeutungen haben,
mit Ausnahme der Verbindungen der Formel (II), in denen gleichzeitig R² und R⁴ jeweils für Wasserstoff, R¹ für Wasserstoff oder C₁-C₅-Alkyl und R³ für Methyl oder Benzyl stehen, sowie weiterhin mit Ausnahme der Verbindungen, in denen R¹ für Methyl, R² für Phenylcarbonyl, R³ für Methyl oder Phenyl, R⁴ für Wasserstoff und Q¹ für Sauerstoff steht.

4. Verfahren zur Herstellung der Carbazate der Formel (II) gemäß Anspruch 3, **dadurch gekennzeichnet, dass** man
a) von C-terminal geschützen Carbazaten der Formel (X) 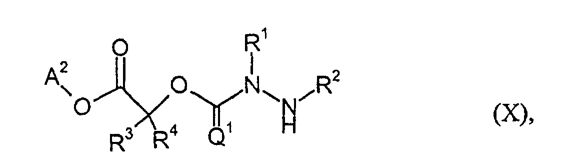 in welcher
R¹, R², R³, R⁴ und Q¹ die in Anspruch 1 angegebene Bedeutung haben und
A² für eine C-terminale Schutzgruppe steht,
in an sich bekannter Weise die Schutzgruppe A² abspaltet oder
b) von N-terminal geschützten Carbazaten der Formel (XI) 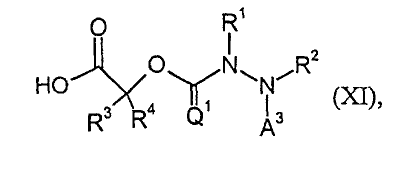 in welcher
R¹, R², R³, R⁴ und Q¹ die in Anspruch 1 angegebene Bedeutung haben und
A³ für eine N-terminale Schutzgruppe steht,
in an sich bekannter Weise die Schutzgruppe A³ abspaltet.

5. C-terminal geschützte Carbazate der Formel (X) 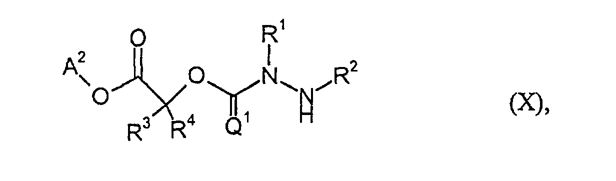 in welcher
R¹, R², R³, R⁴ und Q¹ die in Anspruch 3 angegebene Bedeutung haben und
A² für eine C-terminale Schutzgruppe steht,
wobei die Verbindungen ausgenommen sind in denen R², R⁴ jeweils für Wasserstoff, R¹ für C₁-C₅-Alkyl und R³ für Methyl oder Benzyl stehen, sowie mit Ausnahme der Verbindungen der Formel (X) in denen R¹ für Methyl, R² für Wasserstoff oder Phenylcarbonyl, Q¹ für Sauerstoff, R³ für Phenyl, R⁴ für Wasserstoff und A² für t-Butyl steht, sowie weiterhin mit Ausnahme der Verbindungen, in denen R¹ für Methyl, R² für Phenylcarbonyl, R³ für Methyl, R⁴ für Wasserstoff, Q¹ für Sauerstoff und A² für Benzyl steht.

6. Verfahren zur Herstellung der C-terminal geschützten Carbazate der Formel (X) gemäß Anspruch 5 **dadurch gekennzeichnet, dass** man von N- und O-terminal geschützten Carbazaten der Formel (XII) 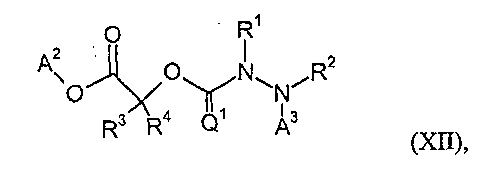 in welcher
R¹, R², R³, R⁴, Q¹ und A² die in Anspruch 5 angegebene Bedeutung haben und A³ die in Anspruch 4 angegebene Bedeutung hat,
in an sich bekannter Weise die N-terminale Schutzgruppe A³ abspaltet.

7. N- und C-terminal geschützte Carbazate der Formel (XII) 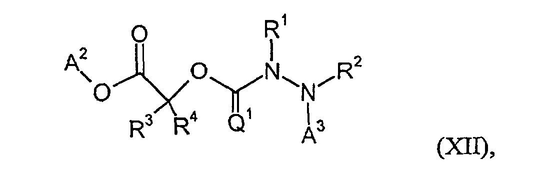 in welcher
R¹, R², R³, R⁴, Q¹ und A² die in Anspruch 5 angegebene Bedeutung haben und A³ die in Anspruch 4 angegebene Bedeutung hat,
wobei die Verbindungen ausgenommen sind, in denen R², R⁴ jeweils für Wasserstoff, R¹ für C₁₋₅-Alkyl und R³ für Methyl oder Benzyl stehen, sowie mit Ausnahme der Verbindungen in denen gleichzeitig R¹ für Methyl, R² für Wasserstoff, R³ für Phenyl, R⁴ für Wasserstoff, Q für Sauerstoff, A² für t-Butyl und A³ für Phenylcarbonyl oder Benzylcarbonyl steht.

8. Verfahren zur Herstellung der N- und C-terminal geschützten Carbazate der Formel (XII) gemäß Anspruch 7
**dadurch gekennzeichnet, dass** man Verbindungen der Formel (XIII) 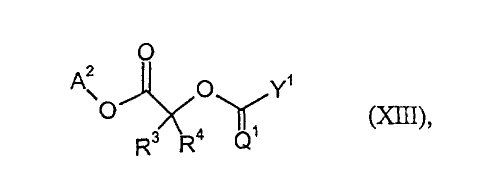 in welcher
A², R³, R⁴ und Q¹ die in Anspruch 7 angegebene Bedeutung haben,
Y¹ für Halogen, Trichlormethoxy, C1-C4-Alkoxy, 1-Imidazolyl oder 1,2,4-Triazolyl steht,
mit Hydrazinen der Formel (XIV) 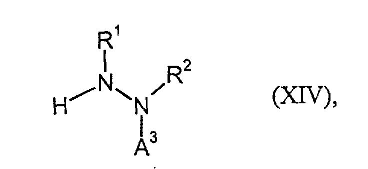 in welcher
R¹, R² und A³ die in Anspruch 7 angegebene Bedeutung haben,
umsetzt.

9. Schädlingsbekämpfungsmittel, **gekennzeichnet durch** einen Gehalt an mindestens einem 1,3,4-Oxadiazin-Derivat der Formel (I) gemäß Anspruch 1.

10. Verwendung von 1,3,4-Oxadiazin-Derivat der Formel (I) gemäß Anspruch 1 zur Bekämpfung von phytopathogenen Schädlingen.

11. Verfahren zur Bekämpfung von phytopathogenen Schädlingen, **dadurch gekennzeichnet, dass** man 1,3,4-Oxadiazin-Derivate der Formel (I) gemäß Anspruch 1 auf phytopathogene Schädlinge und/oder ihren Lebensraum einwirken lässt.

12. Verfahren zur Herstellung von Schädlingsbekämpfungsmitteln und Mitteln zur Bekämpfung von Endoparasiten, **dadurch gekennzeichnet, dass** man 1,3,4-Oxadiazin-Derivate der Formel (I) gemäß Anspruch 1 mit Streckmitteln und/oder oberflächenaktiven Mitteln vermischt.

13. Verwendung von 1,3,4-Oxadiazin-Derivaten der Formel (I) gemäß Anspruch 1 zur Herstellung von Schädlingsbekämpfungsmitteln, sowie Mitteln zur Bekämpfung von Endoparasiten.

## Claims

1. Oxadiazine derivatives of the formula (I) 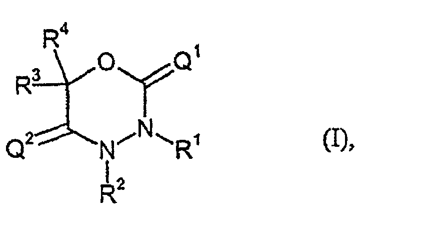 in which
R¹ and R² independently of one another each represent hydrogen, respectively optionally halogen-substituted alkyl, hydroxyalkyl, alkanoyloxyalkyl, alkoxyalkyl, arylalkoxyalkyl, mercaptoalkyl, alkylthioalkyl, alkylsulphinylalkyl, alkylsulphonylalkyl, carboxyalkyl, alkoxycarbonylalkyl, aryloxycarbonylalkyl, arylalkyloxycarbonylalkyl, carbamoylalkyl, aminoalkyl, alkylaminoalkyl, dialkylaminoalkyl, alkoxycarbonylaminoalkyl, alkylcarbonyl, cycloalkylcarbonyl or represent respectively optionally substituted cycloalkyl, cycloalkylalkyl, aryl, arylalkyl, arylcarbonyl, heterocyclylalkyl, hetaryl or hetarylalkyl or
R¹, R² and the two linking nitrogen atoms represent an optionally substituted heterocyclic ring,
R³ and R⁴ independently of one another each represent hydrogen, respectively optionally halogen-substituted alkyl, alkenyl, hydroxyalkyl, alkanoyloxyalkyl, alkoxyalkyl, arylalkoxyalkyl, mercaptoalkyl, alkylthioalkyl, alkylsulphinylalkyl, alkylsulphonylalkyl, alkoxycarbonylalkyl, aryloxycarbonylalkyl, arylalkyloxycarbonylalkyl, carbamoylalkyl, aminoalkyl, alkylaminoalkyl, dialkylaminoalkyl, alkylcarbonyl, cycloalkylcarbonyl or represent respectively optionally substituted cycloalkyl, cycloalkylalkyl, aryl, arylalkyl, hetaryl or hetarylalkyl or
R³ and R⁴ together represent alkylene or the radical (a) 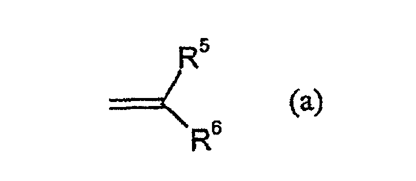 in which
R⁵ and R⁶ independently of one another each represent hydrogen, respectively optionally halogen-substituted alkyl, alkenyl, hydroxyalkyl, alkanoyloxyalkyl, alkoxyalkyl, arylalkoxyalkyl, mercaptoalkyl, alkylthioalkyl, alkylsulphinylalkyl, alkylsulphonylalkyl, alkoxycarbonylalkyl, aryloxycarbonylalkyl, aminoalkyl, alkylaminoalkyl, dialkylaminoalkyl, alkylcarbonyl or represent respectively optionally substituted cycloalkyl, cycloalkylalkyl, aryl, arylalkyl, hetaryl or hetarylalkyl and
Q¹ and Q² independently of one another each represent oxygen or sulphur,
except for 6,6-diphenyl-(1,3,4)-oxadiazinane-2,5-dione.

2. Process for preparing the oxadiazine derivatives of the formula (I) according to Claim 1, 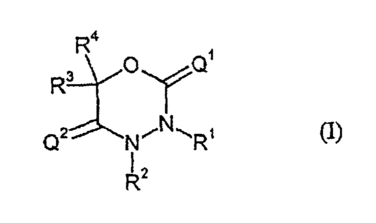 in which
R¹ to R⁴, Q¹ and Q² are each as defined in Claim 1,
**characterized in that**
A) in the case where (1,3,4)-oxadiazine derivatives of the formula (I-a) 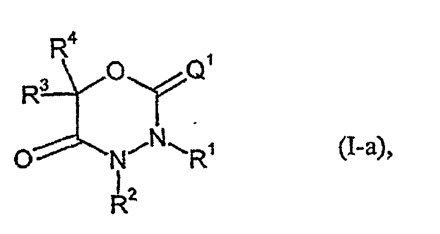 in which
R¹ to R⁴ and Q¹ are each as defined in Claim 1,
are prepared, carbazates of the formula (II) 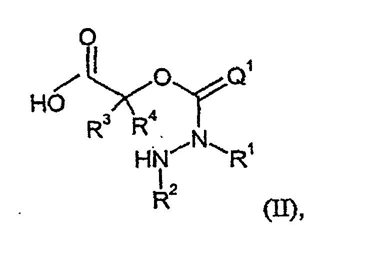 in which
R¹ to R⁴ and Q¹ are each as defined in Claim 1,
are reacted in the presence of a reaction auxiliary and a diluent and, if appropriate, in the presence of a base,
B) in the case where (1,3,4)-oxadiazine derivatives of the formula (I-b) 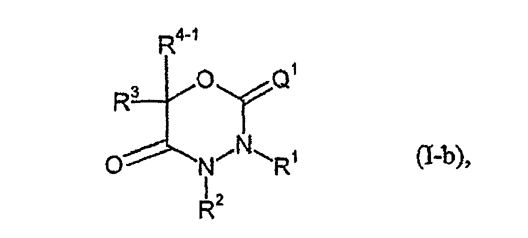 in which
R¹ to R³, Q¹ and Q² are each as defined in Claim 1 and
R⁴⁻¹ represents the same radicals as those defined in Claim 1 for R⁴ with the exception of hydrogen,
are prepared,
(1,3,4)-oxadiazine derivatives of the formula (I-c) 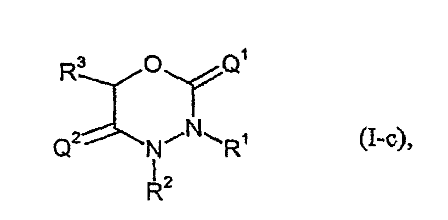 in which
R¹ and R² are each are defined in Claim 1 except hydrogen,
R³, Q¹ and Q² are each as defined in Claim 1,
are reacted with compounds of the formula (III)
R⁴⁻¹ - E (III),
in which
R⁴⁻¹ is as defined in Claim 1 and
E represents an electron-withdrawing leaving group,
if appropriate in the presence of a diluent and, if appropriate, in the presence of a reaction auxiliary,
C) in the case where (1,3,4)-oxadiazine derivatives of the formula (I-d) 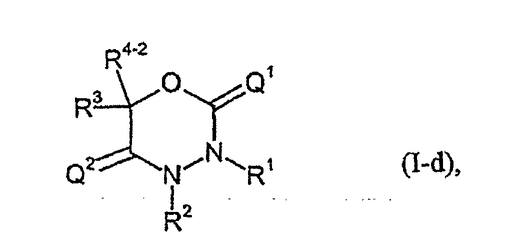 in which
R¹ to R³, Q¹ and Q² are each as defined in Claim 1 and
R⁴⁻² represents the radical (b) 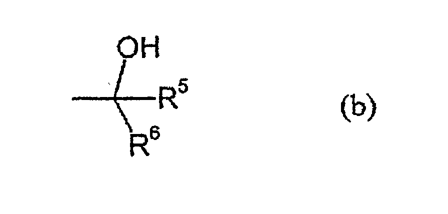 in which
R⁵ and R⁶ independently of one another each represent hydrogen, optionally substituted alkyl or aryl, or
R³ and R⁴⁻² together represent the radical (a) 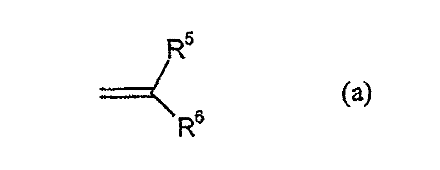 in which
R⁵ and R⁶ are each as defined in Claim 1,
are prepared,
(1,3,4)-oxadiazine derivatives of the formula (I-c) 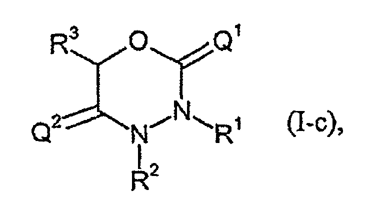 in which
R¹ to R³, Q¹ and Q² are each as defined in Claim 1
are reacted with ketones or aldehdyes of the formula (IV)
R⁵-CO-R⁶ (IV),
in which
R⁵ and R⁶ are each as defined in Claim 1,
if appropriate in the presence of a diluent and, if appropriate, in the presence of a reaction auxiliary, and water is subsequently, if appropriate, eliminated,
D) in the case where (1,3,4)-oxadiazine derivatives of the formula (I-e) 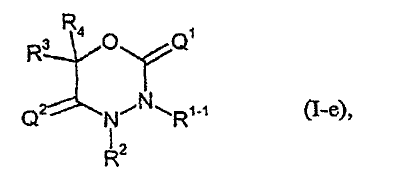 in which
R¹⁻¹ represents the same radicals as those defined in Claim 1 for R¹, with the exception of hydrogen,
R² to R⁴, Q¹ and Q² are each as defined in Claim 1,
are prepared, (1,3,4)-oxadiazine derivatives of the formula (I-f) 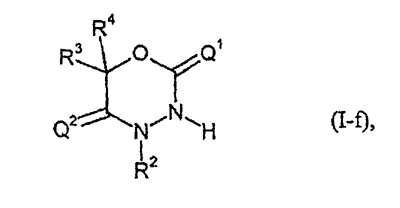 in which
R² to R⁴, Q¹ and Q² are each as defined in Claim 1,
are reacted with compounds of the formula (V)
R¹⁻¹ - E (V),
in which
R¹⁻¹ is as defined above and
E represents an electron-withdrawing leaving group,
if appropriate in the presence of a diluent and, if appropriate, in the presence of a reaction auxiliary,
E) in the case where (1,3,4)-oxadiazine derivatives of the formula (I-g) 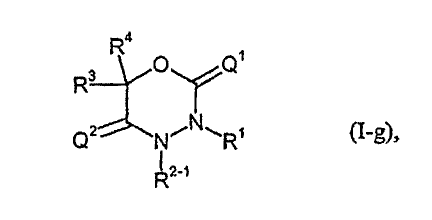 in which
R¹, R³, R⁴, Q¹ and Q² are each as defined in Claim 1 and
R²⁻¹ represents the same radicals as those defined in Claim 1 for R², with the exception of hydrogen,
are prepared, (1,3,4)-oxadiazine derivatives of the formula (I-h) 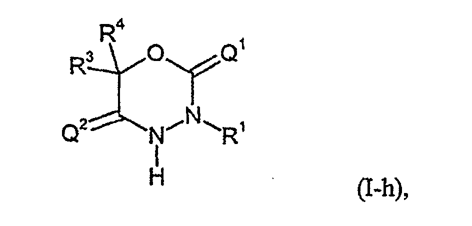 in which
R¹, R³, R⁴, Q¹ and Q² are each as defined in Claim 1,
are reacted with compounds of the formula (VI)
R²⁻¹ - E (VI),
in which
R²⁻¹ is as defined above and
E represents an electron-withdrawing leaving group,
if appropriate in the presence of a diluent and, if appropriate, in the presence of a reaction auxiliary,
F) in the case where (1,3,4)-oxadiazine derivatives of the formula (I) 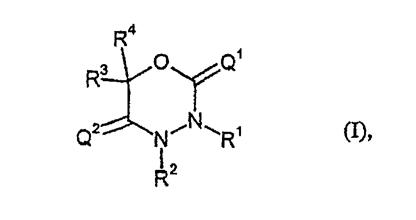 in which
R¹ to R⁴, Q¹ and Q² are each as defined in Claim 1, except for 6,6-diphenyl-(1,3,4)-oxadiazinane-2,5,-dione,
are prepared, compounds of the formula (VII) 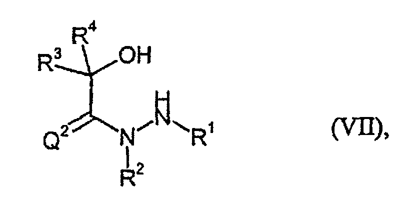 in which
R¹ to R⁴ and Q² are each as defined in Claim 1,
are reacted with compounds of the formula (VIII) 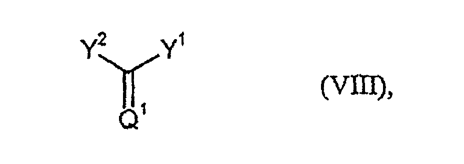 in which
Y¹ represents chlorine, trichloromethoxy, C₁-C₄-alkoxy, optionally substituted phenoxy, 1-imidazolyl or 1,2,4-triazolyl and
Y² represents chlorine, trichloromethoxy, 1-imidazolyl or 1,2,4-triazolyl,
Q¹ is as defined in Claim 1,
if appropriate in the presence of a diluent and, if appropriate, in the presence of a reaction auxiliary and cyclocondensed,
G) in the case where (1,3,4)-oxadiazine derivatives of the formula (I) 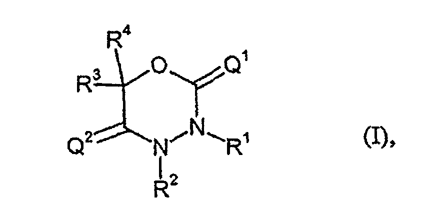 in which
R¹ to R⁴, Q¹ and Q² are each as defined in Claim 1,
are prepared, compounds of the formula (IX) 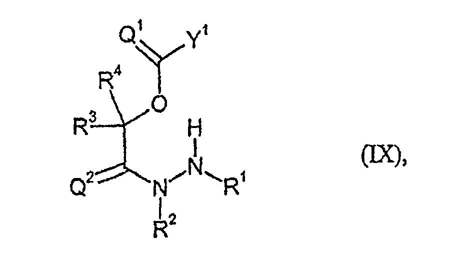 in which
R¹ to R⁴, Q¹, Q² and Y¹ are each as defined in Claim 1 and
are cyclocondensed, if appropriate in the presence of a diluent and, if appropriate, in the presence of a reaction auxiliary,
H) in the case where (1,3,4)-oxadiazine derivatives of the formula (I-i) 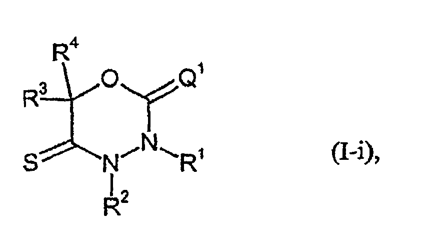 in which
R¹ to R⁴ and Q¹ are each as defined in Claim 1,
are prepared, (1,3,4)-oxadiazine derivatives of the formula (I-a) 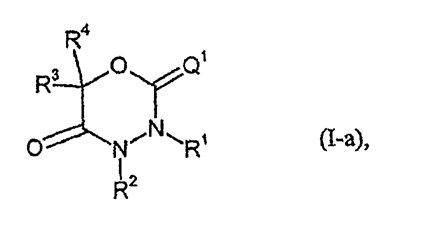 in which
R¹ to R⁴ and Q are each as defined in Claim 1,
are reacted with a thionylating reagent, if appropriate in the presence of a diluent.

3. Carbazates of the formula (II) 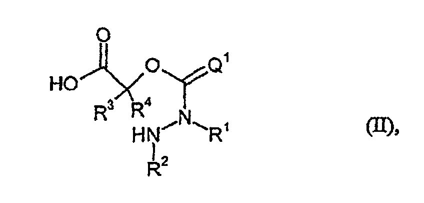 in which
R¹, R², R³, R⁴ and Q¹ are each as defined in Claim 1,
with the exception of the compounds of the formula (II) in which simultaneously R² and R⁴ each represent hydrogen, R¹ represents hydrogen or C₁-C₅-alkyl and R³ represents methyl or benzyl, and furthermore with the exception of compounds in which R¹ represents methyl, R² represents phenylcarbonyl, R³ represents methyl or phenyl, R⁴ represents hydrogen and Q¹ represents oxygen.

4. Process for preparing the carbazates of the formula (II) according to Claim 3, **characterized in that**
a) the protective group A² is cleaved off in a manner known per se from C-terminal protected carbazates of the formula (X) 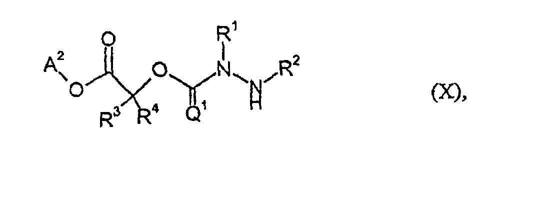 in which
R¹, R², R³, R⁴ and Q¹ are each as defined in Claim 1 and
A² represents a C-terminal protective group,
or
b) the protective group A³ is cleaved off in a manner known per se from N-terminal protected carbazates of the formula (XI) 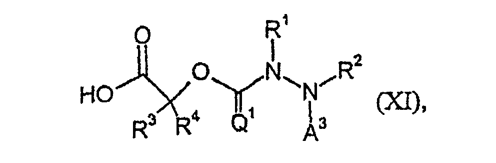 in which
R¹, R², R³, R⁴ and Q¹ are each as defined in Claim 1 and
A³ represents an N-terminal protective group.

5. C-terminal protected carbazates of the formula (X) 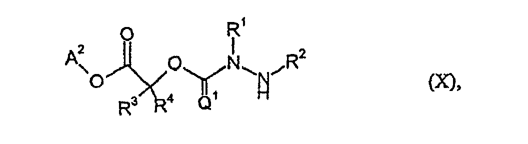 in which
R¹, R², R³, R⁴ and Q¹ are each as defined in Claim 3 and
A² represents a C-terminal protective group,
except for the compounds in which R², R⁴ each represent hydrogen, R¹ represents C₁-C₅-alkyl and R³ represents methyl or benzyl, and with the exception of compounds of the formula (X) in which R¹ represents, methyl, R² represents hydrogen or phenylcarbonyl, Q¹ represents oxygen, R³ represents phenyl, R⁴ represents hydrogen and A² represents tert-butyl, and furthermore with the exception of compounds in which R¹ represents methyl, R² represents phenylcarbonyl, R³ represent methyl, R⁴ represents hydrogen, Q¹ represents oxygen and A² represent benzyl.

6. Process for preparing the C-terminal protected carbazates of the formula (X) according to Claim 5, **characterized in that** the N-terminal protective group A³ is cleaved off in a manner known per se from N- and O-terminal protected carbazates of the formula (XII) 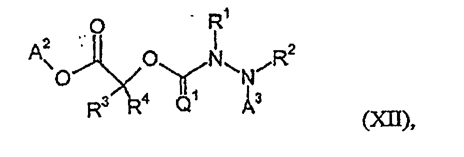 in which
R¹, R², R³, R⁴, Q¹ and A² are each as defined in Claim 5
and A³ is as defined in Claim 4.

7. N- and C-terminal protected carbazates of the formula (XII) 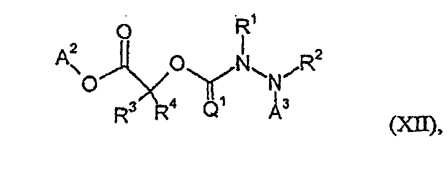 in which
R¹, R², R³, R⁴, Q¹ and A² are each as defined in Claim 5
and A³ is as defined in Claim 4,
except for the compounds in which R², R⁴ each represent hydrogen, R¹ represents C₁₋₅-alkyl and R³ represents methyl or benzyl, and with the exception of compounds in which simultaneously R¹ represents methyl, R² represents hydrogen, R³ represents phenyl, R⁴ represnets hydrogen, Q represents oxygen, A² represents tert-butyl and A³ represents phenylcarbonyl or benzylcarbonyl.

8. Process for preparing the N- and C-terminal protected carbazates of the formula (XII) according to Claim 7,
**characterized in that** compounds of the formula (XIII) 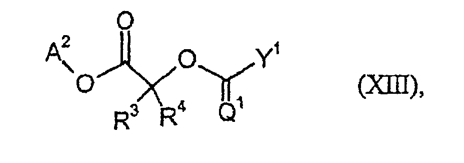 in which
A², R³, R⁴ and Q¹ are each as defined in Claim 7,
Y¹ represents halogen, trichloromethoxy, C₁-C₄-alkoxy, 1-imidazolyl or 1,2,4-triazolyl,
are reacted with hydrazines of the formula (XIV) 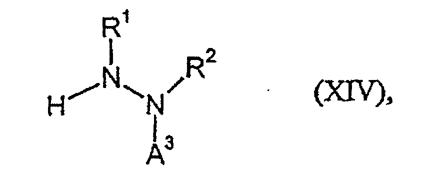 in which
R¹, R² and A³ are each as defined in Claim 7.

9. Pesticides, **characterized in that** they contain at least one 1,3,4-oxadiazine derivative of the formula (I) according to Claim 1.

10. The use of 1,3,4-oxadiazine derivative of the formula (I) according to Claim 1 for controlling phytopathogenic pests.

11. Method for controlling phytopathogenic pests, **characterized in that** 1,3,4-oxadiazine derivatives of the formula (I) according to Claim 1 are allowed to act on phytopathogenic pests and/or their habitat.

12. Process for preparing pesticides and compositions for controlling endoparasites, **characterized in that** 1,3,4-oxadiazine derivatives of the formula (I) according to Claim 1 are mixed with extenders and/or surfactants.

13. The use of 1,3,4-oxadiazine derivatives of the formula (I) according to Claim 1 for preparing pesticides and compositions for controlling endoparasites.

## Revendications

1. Dérivés d'oxadiazine répondant à la formule (I) 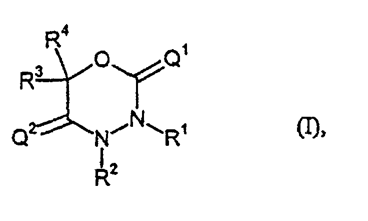 dans laquelle
R¹ et R² représentent, indépendamment l'un de l'autre, un atome d'hydrogène ; un groupe alkyle, un groupe hydroxyalkyle, un groupe alcanoyloxyalkyle, un groupe alcoxyalkyle, un groupe arylalcoxyalkyle, un groupe mercaptoalkyle, un groupe alkylthioalkyle, un groupe alkylsulfinylalkyle, un groupe alkylsulfonylalkyle, un groupe carboxyalkyle, un groupe alcoxycarbonylalkyle, un groupe aryloxycarbonylalkyle, un groupe arylalkyloxycarbonylalkyle, un groupe carbamoylalkyle, un groupe aminoalkyle, un groupe alkylaminoalkyle, un groupe dialkylaminoalkyle, un groupe alcoxycarbonylaminoalkyle, un groupe alkylcarbonyle, un groupe cycloalkylcarbonyle, chacun de ces groupes portant le cas échéant un ou plusieurs substituants halogéno identiques ou différents ; ou représentent un groupe cycloalkyle, un groupe cycloalkylalkyle, un groupe aryle, un groupe arylalkyle, un groupe arylcarbonyle, un groupe hétérocyclylalkyle, un groupe hétaryle ou un groupe hétarylalkyle, chacun de ces groupes étant le cas échéant substitué, ou bien
R¹, R² et les deux atomes d'azote auxquels ils sont liés, représentent un noyau hétérocyclique le cas échéant substitué,
R³ et R⁴ représentent, indépendamment l'un de l'autre, un atome d'hydrogène ; un groupe alkyle, un groupe alcényle, un groupe hydroxyalkyle, un groupe alcanoyloxyalkyle, un groupe alcoxyalkyle, un groupe arylalcoxyalkyle, un groupe mercaptoalkyle, un groupe alkylthioalkyle, un groupe alkylsulfinylalkyle, un groupe alkylsulfonylalkyle, un groupe alcoxycarbonylalkyle, un groupe aryloxycarbonylalkyle, un groupe arylalkyloxycarbonylalkyle, un groupe carbamoylalkyle, un groupe aminoalkyle, un groupe alkylaminoalkyle, un groupe dialkylaminoalkyle, un groupe alkylcarbonyle, un groupe cycloalkylcarbonyle, chacun de ces groupes portant le cas échéant un ou plusieurs substituants halogéno identiques ou différents ; ou représentent un groupe cycloalkyle, un groupe cycloalkylalkyle, un groupe aryle, un groupe arylalkyle, un groupe hétaryle ou un groupe hétarylalkyle, chacun de ces groupes étant le cas échéant substitué, ou bien
R³ et R⁴ représentent ensemble un groupe alkylène ou encore le radical (a) 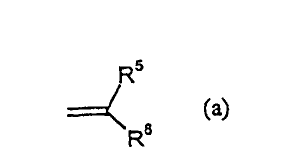
R⁵ et R⁶ représentent, indépendamment l'un de l'autre, un atome d'hydrogène ; un groupe alkyle, un groupe alcényle, un groupe hydroxyalkyle, un groupe alcanoyloxyalkyle, un groupe alcoxyalkyle, un groupe arylalcoxyalkyle, un groupe mercaptoalkyle, un groupe alkylthioalkyle, un groupe alkylsulfinylalkyle, un groupe alkylsulfonylalkyle, un groupe alcoxycarbonylalkyle, un groupe aryloxycarbonylalkyle, un groupe aminoalkyle, un groupe alkylaminoalkyle, un groupe dialkylaminoalkyle, un groupe alkylcarbonyle, chacun de ces groupes portant le cas échéant un ou plusieurs substituants halogéno identiques ou différents ; ou représentent un groupe cycloalkyle, un groupe cycloalkylalkyle, un groupe aryle, un groupe arylalkyle, un groupe hétaryle ou un groupe hétarylalkyle, chacun de ces groupes étant le cas échéant substitué,
Q¹ et Q² représentent, indépendamment l'un de l'autre, un atome d'oxygène ou un atome de soufre,
la 6,6-diphényl-(1,3,4)-oxadiazane-2,5-dione étant exclue.

2. Procédé pour la préparation des dérivés d'oxadiazine répondant à la formule (I), selon la revendication 1 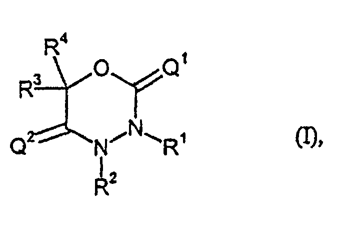 formule dans laquelle
R¹ à R⁴, Q¹ et Q² ont la signification mentionnée à la revendication 1, **caractérisé en ce que**
A) pour le cas où l'on prépare des dérivés de la (1,3,4)-oxadiazine répondant à la formule (I-a) 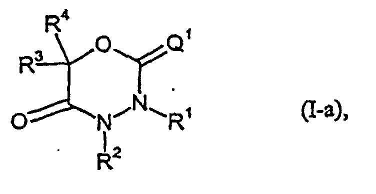 dans laquelle
R¹ à R⁴ et Q¹ ont les significations mentionnées à la revendication 1,
on fait réagir des carbazates répondant à la formule (II) 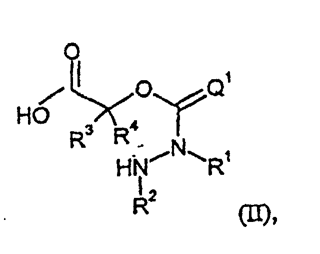 dans laquelle
R¹ à R⁴ et Q¹ ont les significations mentionnées à la revendication 1,
en présence d'un adjuvant de réaction et d'un diluant et, le cas échéant, en présence d'une base,
B) pour le cas où l'on prépare des dérivés de la (1,3,4)-oxadiazine répondant à la formule (I-b) 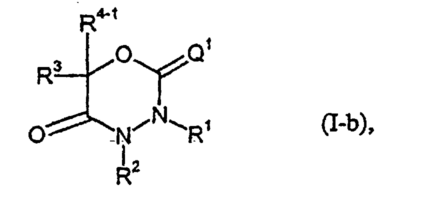 dans laquelle
R¹ à R³ et Q¹ et Q² ont les significations mentionnées à la revendication 1, et
R⁴⁻¹, à l'exception d'un atome d'hydrogène, représente les mêmes radicaux que ceux indiqués dans la revendication 1 pour R⁴,
on fait réagir des dérivés de la (1,3,4)-oxadiazine répondant à la formule (I-c) 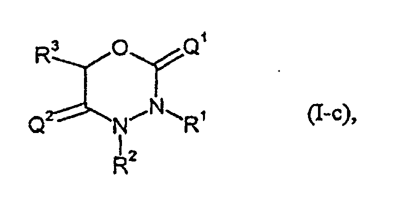 dans laquelle
R¹ et R² ont les significations mentionnées à la revendication 1, à l'exception d'un atome d'hydrogène,
R³, Q¹ et Q² ont les significations mentionnées à la revendication 1,
avec des composés répondant à la formule (III)
R⁴⁻¹ - E (III)
dans laquelle
R⁴⁻¹ a la signification indiquée à la revendication 1, et
E représente un groupe qui se sépare attirant les électrons
le cas échéant en présence d'un diluant et le cas échéant en présence d'un adjuvant de réaction,
C) pour le cas où l'on prépare des dérivés de la (1,3,4)-oxadiazine répondant à la formule (I-d) 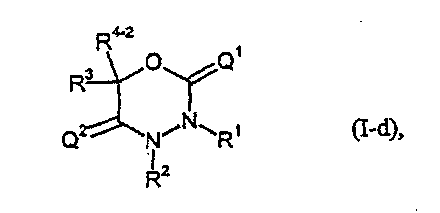 dans laquelle
R¹ à R³, Q¹ et Q² ont les significations mentionnées à la revendication 1, et
R⁴⁻² représente le radical (b) 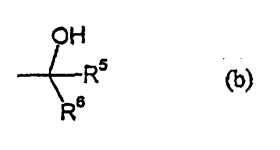 dans lequel
R⁵ et R⁶ représentent, indépendamment l'un de l'autre, un atome d'hydrogène ; un groupe alkyle ou un groupe aryle le cas échéant substitué, ou bien
R³ et R⁴⁻² représentent ensemble le radical (a) 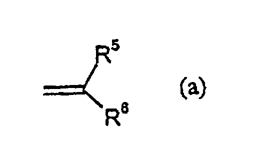 dans lequel
R⁵ et R⁶ ont la signification indiquée à la revendication 1,
on fait réagir des dérivés de la (1,3,4)-oxadiazine répondant à la formule (I-c) 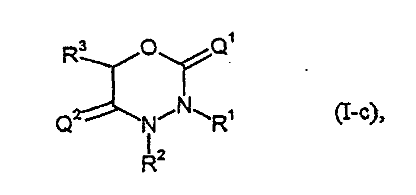 dans laquelle
R¹ à R³, Q¹ et Q² ont les significations mentionnées à la revendication 1,
avec des cétones ou des aldéhydes répondant à la formule (IV)
R⁵-CO-R⁶ (IV)
dans laquelle
R⁵ et R⁶ ont la signification indiquée à la revendication 1,
le cas échéant, en présence d'un diluant et, le cas échéant, en présence d'un adjuvant de réaction et, le cas échéant, on élimine ensuite l'eau,
D) pour le cas où l'on prépare des dérivés de la (1,3,4)-oxadiazine répondant à la formule (I-e) 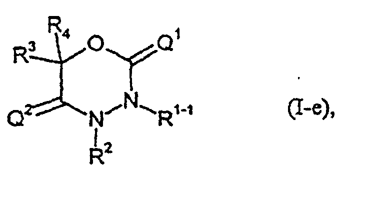 dans laquelle
R¹⁻¹, à l'exception d'un atome d'hydrogène, représente les mêmes radicaux que ceux indiqués dans la revendication 1 pour R¹,
R² à R⁴, Q¹ et Q² ont les significations mentionnées à la revendication 1,
on fait réagir des dérivés de la (1,3,4)-oxadiazine répondant à la formule (I-f) 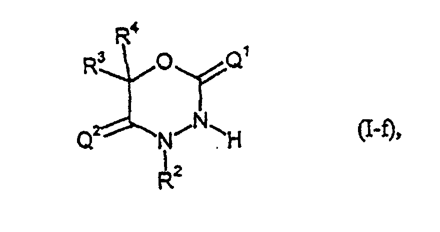 dans laquelle
R² à R⁴, Q¹ et Q² ont les significations mentionnées à la revendication 1,
avec des composés répondant à la formule (V)
R¹⁻¹ - E (V)
dans laquelle
R¹⁻¹ a la signification indiquée ci-dessus, et
E représente un groupe qui se sépare attirant les électrons
le cas échéant en présence d'un diluant et le cas échéant en présence d'un adjuvant de réaction,
E) pour le cas où l'on prépare des dérivés de la (1,3,4)-oxadiazine répondant à la formule (I-g) 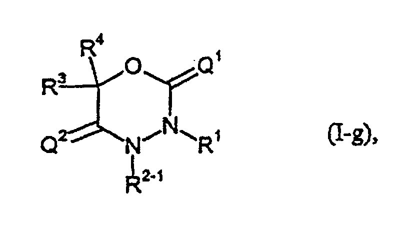 dans laquelle
R¹, R³, R⁴, Q¹ et Q² ont les significations mentionnées à la revendication 1, et
R²⁻¹, à l'exception d'un atome d'hydrogène, représente les mêmes radicaux que ceux indiqués dans la revendication 1 pour R²,
on fait réagir des dérivés de la (1,3,4)-oxadiazine répondant à la formule (I-h) 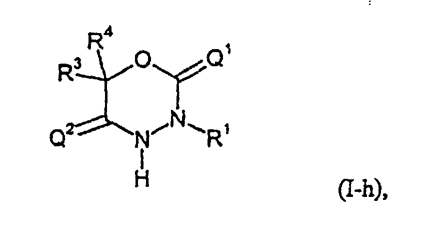 dans laquelle
R¹, R³, R⁴, Q¹ et Q² ont les significations mentionnées à la revendication 1,
avec des composés répondant à la formule (VI)
R²⁻¹ - E (VI)
dans laquelle
R²⁻¹ a la signification indiquée ci-dessus, et
E représente un groupe qui se sépare attirant les électrons
le cas échéant en présence d'un diluant et le cas à échéant en présence d'un adjuvant de réaction,
F) pour le cas où l'on prépare des dérivés de la (1,3,4)-oxadiazine répondant à la formule (I) 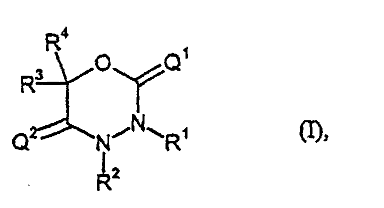 dans laquelle
R¹ à R⁴, Q¹ et Q² ont les significations mentionnées à la revendication 1, la 6,6-diphényl-(1,3,4)-oxadiazane-2,5-dione étant exclue,
on fait réagir des composés répondant à la formule (VII) 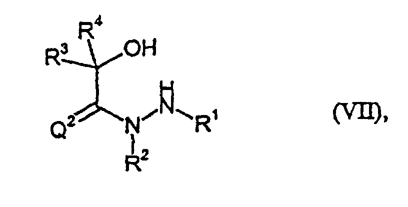 dans laquelle
R¹ à R⁴ et Q² ont les significations mentionnées à la revendication 1,
avec des composés répondant à la formule (VIII) 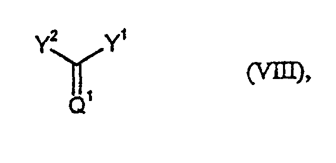 dans laquelle
Y¹ représente un atome de chlore, un groupe trichlorométhoxy, un groupe alcoxy en C₁-C₄, un groupe phénoxy le cas échéant substitué, un groupe 1-imidazolyle ou un groupe 1,2,4-triazolyle, et
Y² représente un atome de chlore, un groupe trichlorométhoxy, un groupe 1-imidazolyle ou un groupe 1,2,4-triazolyle,
Q¹ a la signification indiquée à la revendication 1,
le cas échéant en présence d'un diluant et le cas échéant en présence d'un adjuvant de réaction, avant de procéder à une cyclocondensation,
G) pour le cas où l'on prépare des dérivés de la (1,3,4)-oxadiazine répondant à la formule (I) 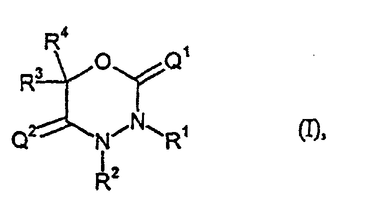 dans laquelle
R¹ à R⁴, Q¹ et Q² ont les significations mentionnées à la revendication 1,
on soumet à une cyclocondensation des composés répondant à la formule (IX) 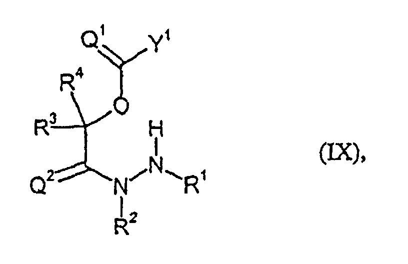 dans laquelle
R¹ à R⁴, Q¹, Q² et Y¹ ont les significations mentionnées à la revendication 1,
le cas échéant, en présence d'un diluant et, le cas échéant, en présence d'un adjuvant de réaction,
H) pour le cas où l'on prépare des dérivés de la (1,3,4)-oxadiazine répondant à la formule (I-i) 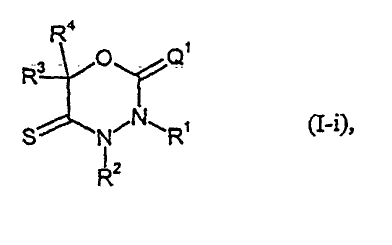 dans laquelle
R¹ à R⁴ et Q¹ ont les significations mentionnées à la revendication 1,
on fait réagir des dérivés de la (1,3,4)-oxadiazine répondant à la formule (I-a) 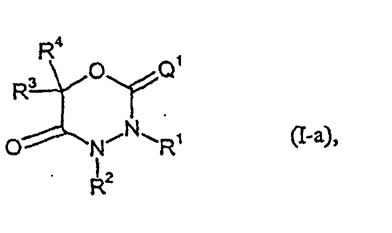 dans laquelle
R¹ à R⁴ et Q ont les significations mentionnées à la revendication 1,
avec un réactif de thionylation, le cas échéant, en présence d'un diluant.

3. Carbazates répondant à la formule (II) 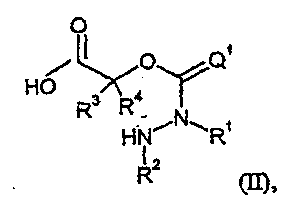 dans laquelle
R¹, R², R³, R⁴ et Q¹ ont les significations mentionnées à la revendication 1,
à l'exception des composés répondant à la formule (II) dans lesquels, de manière simultanée, R² et R⁴ représentent respectivement un atome d'hydrogène, R¹ représente un atome d'hydrogène ou un groupe alkyle en C₁-C₅ et R³ représente un groupe méthyle ou un groupe benzyle, et en outre à l'exception des composés dans lesquels R¹ représente un groupe méthyle, R² représente un groupe phénylcarbonyle, R³ représente un groupe méthyle ou un groupe phényle, R⁴ représente un atome d'hydrogène et Q¹ représente un atome d'oxygène.

4. Procédé pour la préparation des carbazates répondant à la formule (II) selon la revendication 3, **caractérisé en ce qu'**on élimine,
a) de carbazates protégés à leur extrémité C-terminale répondant à la formule (X) 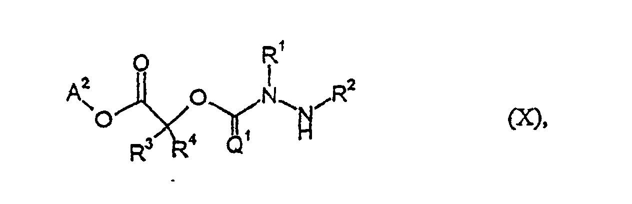 dans laquelle
R¹, R², R³, R⁴ et Q¹ ont les significations mentionnées à la revendication 1, et
A² représente un groupe de protection de l'extrémité C-terminale,
le groupe de protection A² d'une manière connue en soi ou,
b) de carbazates protégés à leur extrémité N-terminale répondant à la formule (XI) 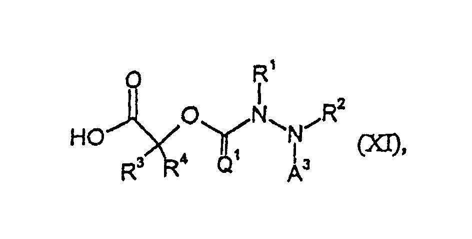
dans laquelle
R¹, R², R³, R⁴ et Q¹ ont la signification mentionnée à la revendication 1, et
A³ représente un groupe de protection de l'extrémité N-terminale,
le groupe de protection A³ d'une manière connue en soi.

5. Carbazates protégés à leur extrémité C-terminale, répondant à la formule (X) 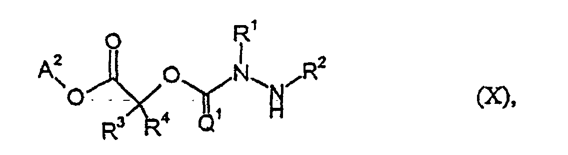 dans laquelle
R¹, R², R³, R⁴ et Q¹ ont les significations mentionnées à la revendication 3 et
A² représente un groupe de protection de l'extrémité C-terminale,
à l'exception des composés dans lesquels, de manière simultanée, R² et R⁴ représentent respectivement un atome d'hydrogène, R¹ représente un groupe alkyle en C₁-C₅ et R³ représente un groupe méthyle ou un groupe benzyle, et en outre à l'exception des composés répondant à la formule (X) dans lesquels R¹ représente un groupe méthyle, R² représente un atome d'hydrogène ou un groupe phénylcarbonyle, Q¹ représente un atome d'oxygène, R³ représente un groupe phényle, R⁴ représente un atome d'hydrogène et A² représente un groupe tert.-butyle, et également à l'exception des composés dans lesquels R¹ représente un groupe méthyle, R² représente un groupe phénylcarbonyle, R³ représente un groupe méthyle, R⁴ représente un atome d'hydrogène, Q¹ représente un atome d'oxygène et A² représente un groupe benzyle.

6. Procédé pour la préparation des carbazates protégés à leur extrémité C-terminale, répondant à la formule (X) selon la revendication 5, **caractérisé en ce qu'**on élimine, de carbazates protégés à leur extrémité N-terminale et à leur extrémité O-terminale, répondant à la formule (XII) 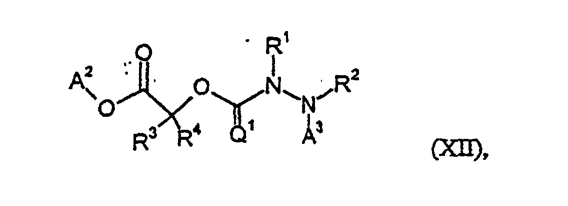 dans laquelle
R¹, R², R³, R⁴, Q¹ et A² ont les significations mentionnées à la revendication 5 et A³ possède la signification indiquée à la revendication 4,
le groupe de protection A³ de l'extrémité N-terminale d'une manière connue en soi.

7. Carbazates protégés à leur extrémité N-terminale et à leur extrémité O-terminale, répondant à la formule (XII) 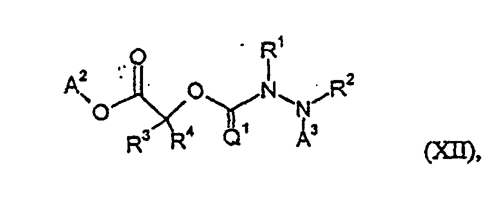 dans laquelle
R¹, R², R³, R⁴, Q¹ et A² ont les significations mentionnées à la revendication 5 et A³ possède la signification indiquée à la revendication 4,
à l'exception des composés dans lesquels R² et R⁴ représentent respectivement un atome d'hydrogène, R¹ représente un groupe alkyle en C₁-C₅ et R³ représente un groupe méthyle ou un groupe benzyle, et en outre à l'exception des composés dans lesquels, de manière simultanée, R¹ représente un groupe méthyle, R² représente un atome d'hydrogène, R³ représente un groupe phényle, R⁴ représente un atome d'hydrogène, Q représente un atome d'oxygène, A² représente un groupe tert.-butyle et A³ représente un groupe phénylcarbonyle ou un groupe benzylcarbonyle.

8. Procédé pour la préparation des carbazates protégés à leur extrémité N-terminale et à leur extrémité C-terminale, répondant à la formule (XII) selon la revendication 7,
**caractérisé en ce qu'**on fait réagir des composés répondant la formule (XIII) 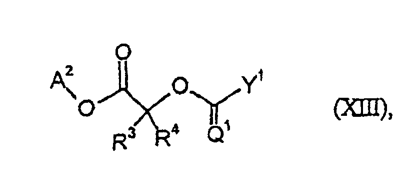 dans laquelle
A², R³, R⁴ et Q¹ xont la signification mentionnée à la revendication 7,
Y¹ représente un atome d'halogène, un groupe trichlorométhoxy, un groupe alcoxy en C₁-C₄, un groupe 1-imidazolyle ou un groupe 1,2,4-triazolyle,
avec des hydrazines répondant à la formule (XIV) 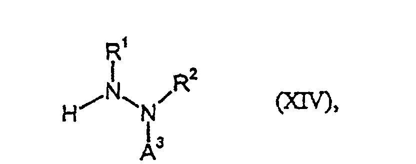 dans laquelle
R¹, R² et A³ ont la signification mentionnée à la revendication 7.

9. Agents de lutte contre les parasites, **caractérisés par** une teneur en au moins un dérivé de la 1,3,4-oxadiazine répondant à la formule (I) selon la revendication 1.

10. Utilisation d'un dérivé de la 1,3,4-oxadiazine répondant à la formule (I) selon la revendication 1, pour lutter contre des parasites phytopathogènes.

11. Procédé pour lutter contre des parasites phytopathogènes, **caractérisé en ce qu'**on laisse agir des dérivés de la 1,3,4-oxadiazine répondant à la formule (I) selon la revendication 1 sur des parasites phytopathogènes et/ou sur leur biotope.

12. Procédé pour la préparation d'agents de lutte contre les parasites et d'agents pour lutter contre les endoparasites, **caractérisé en ce qu'**on mélange des dérivés de la 1,3,4-oxadiazine répondant la formule (I) selon la revendication 1, avec des diluants et/ou avec des agents tensioactifs.

13. Utilisation de dérivés de la 1,3,4-oxadiazine répondant à la formule (I) selon la revendication 1, pour la préparation d'agents de lutte contre des parasites, et d'agents pour lutter contre des endoparasites.
